(19) Europäisches Patentamt
European Patent Office
Office européen des brevets

(11) EP 4 578 848 A1

(12) **EUROPEAN PATENT APPLICATION**
published in accordance with Art. 153(4) EPC

(43) Date of publication:
**02.07.2025 Bulletin 2025/27**

(21) Application number: **23857404.0**

(22) Date of filing: **24.08.2023**

(51) International Patent Classification (IPC):
***C07C 209/88** (2006.01)* ***B01D 15/16** (2006.01)*
***B01D 15/38** (2006.01)* ***B01D 15/40** (2006.01)*
***B01J 20/29** (2006.01)* ***C07B 57/00** (2006.01)*
***C07C 211/27** (2006.01)* ***C07D 209/20** (2006.01)*
***G01N 30/72** (2006.01)*

(52) Cooperative Patent Classification (CPC):
**B01D 15/16; B01D 15/38; B01D 15/40; B01J 20/29; C07B 57/00; C07C 209/88; C07C 211/27; C07D 209/20; G01N 30/72**

(86) International application number:
**PCT/JP2023/030453**

(87) International publication number:
**WO 2024/043296 (29.02.2024 Gazette 2024/09)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.08.2022 JP 2022134509**

(71) Applicant: **Daicel Corporation**
**Osaka-shi, Osaka 530-0011 (JP)**

(72) Inventors:
- **SHIBATA, Toru**
**Tokyo 108-8230 (JP)**
- **SHINKURA, Satoshi**
**Tokyo 108-8230 (JP)**
- **KAWAHARA, Nobuhiro**
**Tokyo 108-8230 (JP)**
- **FUJIOKA, Yuichi**
**Tokyo 108-8230 (JP)**
- **TERADA, Miyu**
**Tokyo 108-8230 (JP)**

(74) Representative: **Grünecker Patent- und Rechtsanwälte**
**PartG mbB**
**Leopoldstraße 4**
**80802 München (DE)**

(54) **AMINE SEPARATION METHOD**

(57) Provided is an amine separation method that includes separating an amine through chromatography using a stationary phase including a ligand having a crown ether-like cyclic structure and being supported on a carrier; and a mobile phase containing a salt of a cation and an acid anion at a concentration of 0.2 mM or more and 50.0 mM or less and containing a solvent having a water content of 50 vol.% or less.

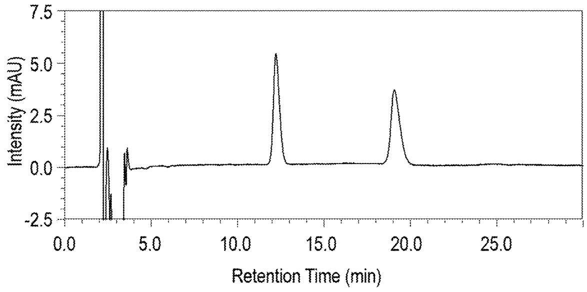


FIG. 3

EP 4 578 848 A1

## Description

TECHNICAL FIELD

The present disclosure relates to a method for separating an amine.

BACKGROUND ART

Separating agents having a crown ether-like cyclic structure are widely used as a stationary phase for liquid chromatography to separate compounds having a primary amino group and similar substances thereof. In particular, it is known that a separating agent in which a crown ether-like cyclic structure is bonded to a chiral structure is useful for separating enantiomers, and is also effective for separating a mixture of multiple compounds having mutually similar structures (for example, a mixture of regioisomers).

Patent Document 1 and Patent Document 2 each disclose, as an excellent separating agent for optical isomers, a separating agent in which a crown ether-like cyclic structure is bonded to a binaphthyl structure of an S-isomer or an R-isomer. Such a separating agent is believed to exhibit separation performance in the following manner. That is, it is thought that a primary ammonium group ($-NH_3^+$) formed by protonation of a primary amine is enclosed in a crown ether-like cyclic structure through hydrogen bonding between the hydrogen atoms of the primary ammonium group and the oxygen atoms of the crown ether-like cyclic structure, and thus the primary ammonium group is retained by the separating agent, and as a result, separation is possible. Therefore, it is known that when such a stationary phase is used, a strongly acidic mobile phase is suitably used to obtain high separation performance. For example, Patent Document 2 discloses a mobile phase obtained by mixing an aqueous perchloric acid solution or an aqueous trifluoroacetic acid solution with an organic solvent such as methanol or acetonitrile. Thus, the strongly acidic mobile phase contains a strong acid. It is known that, among strong acids, perchloric acid most favorably retains an amine and improves separation performance.

However, perchloric acid is not only a strong acid, but also a strong oxidant, and therefore it poses a problem of handling difficulty. Moreover, addition of another strong acid in place of perchloric acid to the mobile phase causes a problem of insufficient retention force of the stationary phase with respect to an amine and an inability to favorably separate the amine.

Meanwhile, Patent Document 3 discloses, as a mobile phase to be combined with a stationary phase having a crown ether-like cyclic structure, a mobile phase containing an aqueous solution of one or more salts of hydrophobic anions selected from the group consisting of salts of chaotropic anions and salts of hydrophobic organic acids. Through the use of such an aqueous mobile phase, the retention of an amine in a column can be strengthened, and the use of a strong acid such as perchloric acid or a strongly oxidizing acid can be avoided.

Non-Patent Literature 1 discloses a mobile phase containing trifluoroacetic acid at a high concentration. This mobile phase can be adapted to liquid chromatography-mass spectrometry (LC-MS).

CITATION LIST

PATENT DOCUMENT

Patent Document 1: JP H2-69472 A
Patent Document 2: WO 2012/050124
Patent Document 3: WO 2020/251003

NON-PATENT LITERATURE

Non-Patent Literature 1: Konya Y, Taniguchi M, Furuno M, Nakano Y, Tanaka N, Fukusaki E, Journal of Chromatography A, 2018, Vol. 1578, pp. 35-44

SUMMARY OF INVENTION

TECHNICAL PROBLEM

Trifluoroacetic acid contained in the mobile phase described in Non-Patent Literature 1 generally has a weak force for retaining an amine in the stationary phase, and satisfactory separation cannot be achieved in many cases. In general, after the use of a high concentration of trifluoroacetic acid, even if a mobile phase containing no trifluoroacetic acid

is used, a so-called ghost peak of trifluoroacetic acid adsorbed on some member of the liquid chromatograph appears, and therefore the use of trifluoroacetic acid is avoided in some cases.

In the method described in Patent Document 3, while it is not necessary to add a strong acid or a strong oxidizing acid to a mobile phase containing a salt of a hydrophobic anion, the solvent contained in the mobile phase is mainly composed of water. Meanwhile, it is not always possible to effectively retain an amine in the stationary phase, even if a salt of a hydrophobic anion is contained in a mobile phase having a low water content.

However, depending on the type of amine to be separated or the purpose of separation, a mobile phase having a low water content may be preferable. Therefore, in order to expand the options of analysis conditions in chromatography, a demand exists for achieving high amine separation performance even when a mobile phase having a low water content is used.

Thus, an object of the present disclosure is to provide a method for separating an amine by chromatography using a stationary phase having a crown ether-like cyclic structure and a mobile phase having a low water content, whereby high separation performance can be achieved.

## SOLUTION TO PROBLEM

As a result of intensive studies to solve the above problems, the present inventors have found that when a specific salt is contained in a mobile phase mainly composed of a nonaqueous solvent, retention of an amine in a stationary phase is promoted, and efficient separation of the amine can be realized. That is, the gist of the present disclosure is as described below.

[1] An amine separation method comprising separating an amine through chromatography using:

a stationary phase comprising a ligand having a crown ether-like cyclic structure and being supported on a carrier; and

a mobile phase comprising a salt of a cation and an acid anion at a concentration of 0.2 mM or more and 50.0 mM or less and comprising a solvent having a water content of 50 vol.% or less.

[2] The amine separation method according to [1], wherein the mobile phase comprises an acid at a concentration of more than 0 mM and 50.0 mM or less.

[3] The amine separation method according to [1] or [2], wherein the solvent has a water content of 20 vol.% or less.

[4] The amine separation method according to any of [1] to [3], wherein a liquid other than water in the solvent is one or more selected from the group consisting of an organic solvent and subcritical or supercritical carbon dioxide.

[5] The amine separation method according to [4], wherein the organic solvent is one or more selected from the group consisting of acetonitrile, acetone, methanol, ethanol, 2-propanol, hexane, tetrahydrofuran, and tert-butyl methyl ether.

[6] The amine separation method according to any of [1] to [3], wherein the solvent comprises acetonitrile in an amount of 70 vol.% or more.

[7] The amine separation method according to any of [1] to [6], wherein an acid from which the acid anion is derived has a $pKa_n$ of -2.0 or more and 6.5 or less in water at 25°C:
wherein, n is a valence of the acid anion.

[8] The amine separation method according to any of [1] to [6], wherein the acid anion is one or more selected from the group consisting of a nitrate ion, a trifluoroacetate ion, an oxalate ion, a hydrogen oxalate ion, a dihydrogen phosphate ion, a glycolate ion, a formate ion, an acetate ion, and a hydrogen carbonate ion.

[9] The amine separation method according to any of [1] to [6], wherein the acid anion is one or more selected from the group consisting of an oxalate ion, a formate ion, and an acetate ion.

[10] The amine separation method according to any of [1] to [9], wherein the cation is one or more selected from the group consisting of a tetrahydroammonium ion, a secondary ammonium ion, and a tertiary ammonium ion.

[11] The amine separation method according to [2], wherein a value calculated by Formula (1) described below for the mobile phase is 0.01 or more and 0.90 or less:
[Math. 1]

$$\frac{qY}{X + qY} \qquad (1)$$

wherein,

X is a concentration (mM) of the acid in the mobile phase;
Y is a concentration (mM) of the salt in the mobile phase; and
q is the number of acid anions per molecule of the salt.

[12] The amine separation method according to any of [1] to [11], wherein the chromatography is carried out by liquid chromatography-mass spectrometry.

ADVANTAGEOUS EFFECTS OF INVENTION

According to the present disclosure, there can be provided a method for separating an amine by chromatography using a stationary phase having a crown ether-like cyclic structure and a mobile phase having a low water content, whereby high separation performance can be achieved.

In a preferred embodiment of the present disclosure, an amine separation method applicable to LC-MS can be provided.

BRIEF DESCRIPTION OF DRAWINGS

FIG. 1 is a liquid chromatogram of dl-1-phenylethylamine in Example 1.
FIG. 2 is a liquid chromatogram of dl-1-phenylethylamine in Example 2.
FIG. 3 is a liquid chromatogram of dl-1-phenylethylamine in Example 3.
FIG. 4 is a liquid chromatogram of dl-1-phenylethylamine in Example 4.
FIG. 5 is a liquid chromatogram of dl-1-phenylethylamine in Example 5.
FIG. 6 is a liquid chromatogram of dl-1-phenylethylamine in Example 6.
FIG. 7 is a liquid chromatogram of dl-1-phenylethylamine in Example 7.
FIG. 8 is a liquid chromatogram of dl-1-phenylethylamine in Example 8.
FIG. 9 is a liquid chromatogram of dl-1-phenylethylamine in Comparative Example 1.
FIG. 10 is a liquid chromatogram of dl-1-phenylethylamine in Comparative Example 2.
FIG. 11 is a liquid chromatogram of dl-1-phenylethylamine in Comparative Example 3.
FIG. 12 is a liquid chromatogram of dl-1-phenylethylamine in Comparative Example 4.
FIG. 13 is a liquid chromatogram of dl-1-phenylethylamine in Comparative Example 5.
FIG. 14 is a liquid chromatogram of dl-1-phenylethylamine in Comparative Example 6.
FIG. 15 is a liquid chromatogram of dl-1-phenylethylamine in Example 9.
FIG. 16 is a liquid chromatogram of dl-1-phenylethylamine in Comparative Example 7.
FIG. 17 is a liquid chromatogram of dl-1-phenylethylamine in Comparative Example 8.
FIG. 18 is a liquid chromatogram of dl-tryptophan in Example 10.
FIG. 19 is a liquid chromatogram of dl-tryptophan in Example 11.
FIG. 20 is a liquid chromatogram of dl-tryptophan in Example 12.
FIG. 21 is a liquid chromatogram of dl-tryptophan in Comparative Example 9.
FIG. 22 is a liquid chromatogram of dl-tryptophan in Comparative Example 10.
FIG. 23 is a liquid chromatogram of dl-tryptophan in Example 13.
FIG. 24 is a graph showing the relationship between retention time and the salt concentration and acid concentration in the mobile phases used in Examples 14 to 16.

DESCRIPTION OF EMBODIMENTS

The present disclosure is described in detail below with reference to specific embodiments. However, each of the configurations in each embodiment, combinations thereof, and the like are merely examples, and various additions, omissions, substitutions, and other changes of the configurations may be made as appropriate within a scope that does not depart from the spirit of the present disclosure. The present disclosure is not limited by the embodiments and is limited only by the claims.

Each embodiment disclosed in the present specification can be combined with any other feature disclosed herein.

**In** the present specification, when lower limit values and upper limit values of a numerical range are separately indicated, the numerical range can be a combination of any lower limit value and any upper limit value among those lower and upper limit values.

[Amine Separation Method]

One embodiment of the present disclosure is an amine separation method that includes a step of separating an amine through chromatography using a stationary phase including a ligand having a crown ether-like cyclic structure and being supported on a carrier (hereinafter, the stationary phase may be referred to as a "stationary phase having a crown ether-like cyclic structure") and a mobile phase containing a salt of a cation and an acid anion (hereinafter may be referred to simply as a "salt") at a concentration of 0.2 mM or more and 50.0 mM or less and containing a solvent having a water content of 50 vol.% or less.

The amine separation method according to the present embodiment may include, besides the above-mentioned separation step, additional steps, such as a step of identifying the separated amine, a step of quantifying the separated amine, and a step of recovering the separated amine.

Firstly, the significance of the present embodiment will be described. The present inventors presumed that when an ammonium group generated by protonation of an amino group of an amine to be separated is included (retained) in a crown ether-like cyclic structure of the stationary phase, an anion present in the mobile phase serves as a counter ion of the ammonium group and is present in the vicinity of the ammonium group. This is a type of adsorption equilibrium reaction. Therefore, in order for the ammonium group to be sufficiently retained by the crown ether-like cyclic structure, the anion serving as a counter ion (hereinafter may be referred to as a "counter anion") must be present at a certain concentration in the mobile phase.

However, generally, acid dissociation does not easily occur in an organic solvent having a low polarity, with the exception of special basic organic solvents. Therefore, when a solvent having a low water content is used as the solvent for the mobile phase, it becomes difficult to supply a necessary amount of the counter anion to the mobile phase. **In** particular, among acids, weak acids are more difficult to dissociate, and thus when the acid contained in the mobile phase is a weak acid, it becomes more difficult to supply a sufficient concentration of counter anions to the mobile phase.

Therefore, the present inventors considered that the use of a solvent having a low water content in a mobile phase containing an acid is not appropriate for separating an amine by using a stationary phase having a crown ether-like cyclic structure. However, as described above, depending on the type of amine to be separated or the purpose of separation, a mobile phase having a low water content may be preferable.

Therefore, the present inventors examined methods for realizing sufficient retention of an amine in a stationary phase even in the case of using a stationary phase having a crown ether-like cyclic structure and a mobile phase having a low water content. As a result, the present inventors discovered that when a base is added to a mobile phase containing an acid and a solvent having a low water content for neutralizing some or all of the acid to convert the acid into a salt, the counter anion concentration in the mobile phase can be adjusted to a preferable level even if the acid is a weak acid. It is considered that when a sufficient amount of counter anions are supplied to the mobile phase in this manner, the ammonium group generated from the amine to be separated forms an ion pair with the counter anion and is subjected to inclusion in the crown ether-like cyclic structure of the stationary phase. Furthermore, it is presumed that when inclusion of the ammonium group in the crown ether-like cyclic structure, i.e., retention of the amine in the stationary phase is promoted, the amine separation performance is improved.

Summarizing the above, the fact that the solvent contained in the mobile phase used in the separation step in the present embodiment is a solvent having a water content of 50 vol.% or less means that a mobile phase having a low water content is used for chromatography in the separation step. In addition, the fact that the mobile phase contains a salt of a cation and an acid anion at a concentration of 0.2 mM or more and 50.0 mM or less means that a salt for supplying an appropriate amount of anions (i.e., acid anions) is contained in the mobile phase, or in other words, that an anion which forms a pair with an ammonium group is sufficiently present in the mobile phase.

In the present embodiment, the amine separation performance can be improved by using a mobile phase obtained by neutralizing some or all of the acid to convert the acid into a salt, or a mobile phase corresponding thereto. Thus, the acid is not an essential component. A mobile phase in which the acid concentration is 0 mM corresponds to an embodiment in which all of the acid is neutralized, whereas a mobile phase in which the acid concentration exceeds 0 mM corresponds to an embodiment in which some of the acid is neutralized.

In the present embodiment, a mobile phase obtained by neutralizing some or all of an acid to convert the acid into a salt, or a mobile phase corresponding thereto, i.e., a mobile phase containing a salt of a cation and an acid anion and containing an acid as necessary exhibits an effect of promoting retention of an amine in the stationary phase and improving amine separation performance. In this case, the method for preparing the mobile phase containing the above components is not particularly limited. As long as the mobile phase contains the above-described components, the amine separation performance can be improved with a mobile phase prepared by any method. Therefore, as described below, the mobile phase may be prepared by a method of neutralizing a liquid containing an acid, or may be prepared by a method of dissolving a preliminarily prepared salt in a solvent and adding an acid as necessary.

In the separation method according to the present embodiment, an amine can be separated with high separation performance by using a mobile phase containing a solvent having a low water content (i.e., a solvent having a water

content of 50 vol.% or less). Therefore, the separation method is particularly useful for separating a separation target that cannot be effectively retained in the stationary phase when a mobile phase containing an aqueous solvent having a high water content is used.

Next, the separation step of the present embodiment will be described in detail.

1. Amine

According to the separation method of the present embodiment, a mixture of a plurality of amines can be separated into individual amines, and an amine can be separated from a mixture containing the amine and a non-amine.

The amine is not particularly limited as long as it is a compound containing an amino group, and may be any of a primary amine, a secondary amine, and a tertiary amine. Specific examples of the amine include amino acids, such as alanine, cysteine, glutamic acid, methionine, leucine, tyrosine, and tryptophan; amino acid derivatives, such as amides and esters of the above-mentioned amino acids; aminoalcohols, such as dimethylaminoethanol, propanolamine, methioninol, and norephedrine; and amino group-containing hydrocarbons, such as phenylethylamine, aniline, methylaniline, chloroaniline, and aminobenzoic acid; and cyclic amines, such as 1-phenyl-1,2,3,4-tetrahydroisoquinoline.

The separation method according to the present embodiment is suitable for separating a desired primary amine from a mixture containing the primary amine. This is because the primary amine is favorably retained by the crown ether-like cyclic structure of the stationary phase.

Furthermore, according to the separation method of the present embodiment, a high level of separation performance can be achieved, and therefore a mixture of amines that are difficult to separate due to their mutually similar structures can be separated into individual amines. Examples of mixtures of amines having mutually similar structures include a mixture of chain isomers, a mixture of regioisomers, a mixture of geometric isomers, and a mixture of analogs. A mixture of enantiomers can be separated into individual enantiomers by using, as the stationary phase, a chiral stationary phase having a crown ether-like cyclic structure into which an optically active site is introduced.

2. Mobile Phase

The mobile phase used in the separation step contains a salt of a cation and an acid anion at a concentration of 0.2 mM or more and 50.0 mM or less and contains a solvent having a water content of 50 vol.% or less.

The mobile phase may contain an additional component as long as the separation performance is not impaired. The mobile phase is preferably a homogeneous system (single-phase system) at least under the conditions in which an analysis is performed.

As used herein, the term "mobile phase" means a liquid which, in chromatography such as liquid chromatography or supercritical fluid chromatography, is poured into the stationary phase together with the separation target, moves together with the separation target, and then elutes a separated substance from the stationary phase.

2-1. Solvent

The solvent contained in the mobile phase in the present embodiment is not particularly limited as long as the water content is 50 vol.% or less. That is, among the solvents contained in the mobile phase, the liquid other than water (hereinafter may be referred to as a "nonaqueous solvent") is not particularly limited. The nonaqueous solvent is preferably one or more selected from the group consisting of an organic solvent (water content: 0 vol.%) and subcritical or supercritical carbon dioxide. Accordingly, examples of the solvent contained in the mobile phase include a mixed solvent of water and an organic solvent, an organic solvent (water content: 0 vol.%), a mixed solvent of subcritical or supercritical carbon dioxide and an organic solvent, and a mixed solvent of subcritical or supercritical carbon dioxide, water, and an organic solvent. When the solvent contained in the mobile phase is a mixed solvent composed of two or more solvents, the two or more solvents are preferably a combination of solvents that do not cause phase separation under the amine separation conditions.

The organic solvent is not particularly limited, but is preferably an organic solvent that can dissolve the amine to be separated. Examples of suitable organic solvents include acetonitrile, acetone, tetrahydrofuran (THF), tert-butyl methyl ether, methanol, ethanol, n-propanol, 2-propanol, tetrahydrofuran, dimethyl sulfoxide (DMSO), dimethylformamide (DMF), and dimethylacetamide (DMAc). The organic solvent may contain a hydrocarbon such as hexane, a halogenated hydrocarbon such as dichloromethane, or the like as long as the organic solvent can dissolve various components, such as an amine to be separated, and a salt of a cation and an acid anion. Among such organic solvents, from the viewpoint of high chemical stability, the organic solvent preferably contains one or more selected from the group consisting of acetonitrile, acetone, methanol, ethanol, 2-propanol, hexane, THF, and tert-butyl methyl ether, and is more preferably acetonitrile. Particularly preferably, the solvent contained in the mobile phase contains acetonitrile in an amount of 70 vol.% or more. The organic solvent may be a single type of organic solvent, or may be a combination of any two or more types of organic

solvents in any proportions.

The organic solvent may be a mixed liquid generally used in chromatography, such as a mixed liquid of a hydrocarbon having 5 or more and 8 or less carbons and an alcohol. Examples of such a mixed liquid include a hexane/ethanol mixed liquid that is compatible with water. In the case of an organic solvent having a hexane/ethanol mixing ratio of, for example, 100/100 (v/v), a mixed solvent having a water content of about 10 vol.% can be prepared.

The mixed liquid of a hydrocarbon having 5 or more and 8 or less carbons and an alcohol has a low viscosity, and therefore a high flow rate can be realized at a low pressure in chromatography. In addition, the ultraviolet absorption is small, and therefore detection of an amine by the ultraviolet absorption can be realized.

When the nonaqueous solvent contains subcritical or supercritical carbon dioxide, chromatography is carried out by a supercritical fluid chromatograph. In this case, the compatibility between a component such as a salt and subcritical or supercritical carbon dioxide is not necessarily good, and therefore subcritical or supercritical carbon dioxide is preferably used in combination with an organic solvent (water content: 0 vol.%) or a mixed solvent of water and an organic solvent as an auxiliary solvent so that the target component can be dissolved in the mobile phase at the target concentration. The term organic solvent used herein has the same meaning as the organic solvent exemplified as a nonaqueous solvent. Preferable examples of the organic solvent and the mixed solvent of water and an organic solvent as the auxiliary solvent are methanol and a mixed solvent of water and methanol, respectively.

When the solvent contained in the mobile phase contains subcritical or supercritical carbon dioxide, the volume of the solvent is calculated based on the density of liquid carbon dioxide at - 20°C, i.e., 1.030 g/mL.

The water content of the solvent contained in the mobile phase is not particularly limited as long as the water content is 50 vol.% or less, but is preferably 40 vol.% or less, more preferably 30 vol.% or less, still more preferably 20 vol.% or less, and particularly preferably 10 vol.% or less. The lower limit of the water content in the solvent contained in the mobile phase is not particularly limited, but is usually 0 vol.% or more, and preferably more than 0 vol.%, 0.5 vol.% or more, or 1.0 vol.% or more. That is, preferable ranges of the water content of the solvent contained in the mobile phase are, for example, 0 vol.% or more and 50 vol.% or less, 0 vol.% or more and 40 vol.% or less, more than 0 vol.% and 30 vol.% or less, 0.5 vol.% or more and 20 vol.% or less, and 1.0 vol.% or more and 10 vol.% or less.

2-2. Salt of Cation and Acid Anion

The salt of a cation and an acid anion contained in the mobile phase used in the separation step is formed from any cation and any acid anion. The salt may be a single type, or may be a combination of any two or more types in any proportions.

**In** the present specification, the anion component forming the salt is referred to as an "acid anion" because it was discovered that amine separation performance can be improved by using a mobile phase obtained by neutralizing some or all of the acid to convert the acid into a salt or by using a mobile phase corresponding thereto. However, as long as the anion component is an anion of the same type as the acid anion, the anion component does not necessarily need to be an anion generated from an acid and may be, for example, an anion derived from cation exchange or the like.

Preferable examples of the cation that forms a salt by bonding with an acid anion include a tetrahydroammonium ion, a primary ammonium ion, a secondary ammonium ion, a tertiary ammonium ion, and a quaternary ammonium ion. However, the cation is not limited thereto, and may be an ion of an alkali metal such as lithium, sodium, or potassium, as well as an ion of another metal. The cation forming the salt may be a single type or may be a combination of any two or more types in any proportions.

Preferable examples of the primary ammonium ion include monoalkylammonium ions such as a methylammonium ion, an ethylammonium ion, a propylammonium ion, and a butylammonium ion.

Preferable examples of the secondary ammonium ion include dialkylammonium ions such as a dimethylammonium ion, a diethylammonium ion, a diisopropylammonium ion, and a dibutylammonium ion; and cyclic secondary ammonium ions such as an N-unsubstituted pyrrolidinium ion and an N-unsubstituted piperidinium ion.

Preferable examples of the tertiary ammonium ion include trialkylammonium ions such as a trimethylammonium ion, a triethylammonium ion, a diisopropylethylammonium, and a tributylammonium ion; and cyclic tertiary ammonium ions such as an N-substituted pyrrolidinium ion, an N-substituted piperidinium ion, and an N-unsubstituted 1,4-diazabicyclo[2.2.2]octane-1,4-diium ion.

Preferable examples of the quaternary ammonium ion include tetraalkylammonium ions such as a tetramethylammonium ion, a tetraethylammonium ion, a tetrabutylammonium ion, a hexadecyltrimethylammonium ion, and an octadecyltrimethylammonium ion; and cyclic quaternary ammonium ions such as an N,N-di-substituted pyrrolidinium ion, an N,N-di-substituted piperidinium ion, and an N,N'-di-substituted-1,4-diazabicyclo[2.2.2]octane-1,4-diium ion.

The alkyl group bonded to the nitrogen atom of each of the monoalkylammonium ion, the dialkylammonium ion, the trialkylammonium ion, and the tetraalkylammonium ion is preferably an alkyl group having 1 or more and 20 or less carbons, more preferably an alkyl group having 1 or more and 10 or less carbons, still more preferably an alkyl group having 1 or more and 4 or less carbons, and particularly preferably an alkyl group having 1 or 2 carbons. Such a primary to tertiary

ammonium ion having a small number of carbons of the alkyl group has a low molecular weight. Therefore, when chromatography is carried out by LC-MS, an unnecessary peak is less likely to occur in the vicinity of the peak of the amine to be separated, and the primary to tertiary ammonium ion is thermally dissociated and easily vaporized, which is preferable.

The substituent bonded to the nitrogen atom of each of the N-substituted pyrrolidinium ion, the N-substituted piperidinium ion, the N,N-di-substituted pyrrolidinium ion, the N,N-di-substituted piperidinium ion, and the N,N'-di-substituted-1,4-diazabicyclo[2.2.2]octane-1,4-diium ion is not particularly limited, but is preferably an alkyl group having 1 or more and 20 or less carbons, more preferably an alkyl group having 1 or more and 10 or less carbons, still more preferably an alkyl group having 1 or more and 4 or less carbons, and particularly preferably an alkyl group having 1 or 2 carbons. This is because, as described above, the molecular weight of the ammonium ion is preferably small in LC-MS.

The cation is preferably one or more selected from the group consisting of a tetrahydroammonium ion, a secondary ammonium ion, and a tertiary ammonium ion. That is, the salt is preferably one or more selected from the group consisting of a tetrahydroammonium salt, a secondary ammonium salt, and a tertiary ammonium salt. Such an ammonium salt is less likely to interact with the crown ether-like cyclic structure of the stationary phase, and therefore the risk of inhibiting retention of the amine to be separated is small, and the amine separation performance can be further improved.

Particularly suitable cations include one or more cations selected from the group consisting of a secondary ammonium ion and a tertiary ammonium ion. Among these, the cation is particularly preferably a cation having a low molecular weight, specifically, a dimethylammonium ion, a diethylammonium ion, a trimethylammonium ion, a triethylammonium ion, or the like. That is, the salt is more preferably one or more selected from the group consisting of a secondary ammonium salt and a tertiary ammonium salt, and is even further preferably a salt having a low molecular weight, such as a dimethylammonium salt, a diethylammonium salt, a trimethylammonium salt, or a triethylammonium salt. A secondary ammonium salt and a tertiary ammonium salt are easily thermally dissociated and volatilized. Therefore, when the separation step is carried out by LC-MS, the risk of a secondary ammonium salt or a tertiary ammonium salt being deposited in the detector and clogging the nozzle or contaminating the detector is minimal. In addition, when the molecular weight is low, the risk of generating an unnecessary peak in the vicinity of the peak of the amine to be separated when LC-MS is carried out as described above can be reduced.

The acid from which the acid anion is derived is not particularly limited, and may be one or more known acids selected from organic acids and inorganic acids. Such a known acid is an acid that generates the above-mentioned acid anion by dissociation, or in other words, is, in terms of the electric charge, a neutral acid that is obtained by protonating the acid anion. The upper limit of the valence of the acid anion is not particularly limited, but is an integer of usually 6 or less, preferably 4 or less, and more preferably 2 or less. Table 1 below shows an example of the relationship between the acid anion, the valence of the acid anion, and the acid from which the acid anion is derived in the present embodiment. As shown in Table 1, a plurality of types of acid anions having different valences may be generated from the same acid. The acid anion forming the salt may be a single type or may be a combination of any two or more types in any proportions.

[Table 1]

Table 1

| Acid anion | | Valence of acid anion | Acid from which acid anion is derived | |
|---|---|---|---|---|
| Formate ion | $HCOO^-$ | 1 | Formic acid | $HCOOH$ |
| Oxalate ion | $C_2O_4^{2-}$ | 2 | Oxalic acid | $(COOH)_2$ |
| Hydrogen oxalate ion | $HC_2O_4^-$ | 1 | Oxalic acid | $(COOH)_2$ |
| Phosphate ion | $PO_4^{3-}$ | 3 | Phosphoric acid | $H_3PO_4$ |
| Monohydrogen phosphate ion | $HPO_4^{2-}$ | 2 | Phosphoric acid | $H_3PO_4$ |
| Dihydrogen phosphate ion | $H_2PO_4^-$ | 1 | Phosphoric acid | $H_3PO_4$ |

The acid anion is preferably derived from an acid having a specific pKa. Specifically, the $pKa_n$ (n is the valence of the acid anion) of the acid from which the acid anion is derived is preferably -2.9 or more, more preferably -2.0 or more, even more preferably -1.0 or more, particularly preferably 0.2 or more, and most preferably 1.0 or more, and is preferably 6.5 or less, more preferably 6.0 or less, even more preferably 5.5 or less, particularly preferably 4.5 or less, and most preferably 3.5 or less. That is, preferred ranges of the $pKa_n$ of the acid from which the acid anion is derived are, for example, -2.9 or more and 6.5 or less, -2.0 or more and 6.5 or less, -1.0 or more and 6.0 or less, 0.2 or more and 5.5 or less, and 1.0 or more

and 4.5 or less. Alternatively, preferred ranges of the $pKa_n$ of the acid from which the acid anion is derived are, for example, -2.9 or more and 6.5 or less, -2.0 or more and 6.0 or less, -1.0 or more and 5.5 or less, 0.2 or more and 4.5 or less, and 1.0 or more and 3.5 or less.

The present inventors presume that the reason why an acid anion derived from an acid having a $pKa_n$ value of -2.9 or more is preferable as the acid anion is as follows.

When the ammonium group is included in the crown ether-like cyclic structure of the stationary phase, at least partial desolvation is considered to be necessary, but in general, in a weakly polar solvent, an acid anion derived from a weak acid is not sufficiently stabilized by solvation. Therefore, it is presumed that the energy loss of a weak acid due to desolvation when the ammonium group is included in the crown ether-like cyclic structure of the stationary phase is small. However, a weak acid cannot produce a sufficient concentration of a conjugate anion in an organic solvent. In contrast, in the present embodiment, it is presumed that retention of the amine in the stationary phase is promoted because the mobile phase contains a salt of a cation and an acid anion derived from a weak acid having a $pKa_n$ of -2.9 or more.

In the present specification, unless otherwise specified, the acid dissociation constant "pKa" of the acid means the acid dissociation constant of the acid in water at 25°C.

Preferable examples of the acid having a $pKa_n$ as described above include methanesulfonic acid ($pKa_1$ = -2.6), nitric acid ($pKa_1$ = -1.4), trifluoroacetic acid ($pKa_1$ = 0.23), oxalic acid ($pKa_1$ = 1.27, $pKa_2$ = 4.27), phosphoric acid ($pKa_1$ = 2.12), glycolic acid ($pKa_1$ = 3.1), formic acid ($pKa_1$ = 3.75), acetic acid ($pKa_1$ = 4.76), and carbonic acid ($pKa_1$ = 3.60). Among these, the acid is more preferably one or more selected from the group consisting of formic acid, oxalic acid, and acetic acid, and is even more preferably oxalic acid, from the viewpoint of less noise in detection by a mass detector when the separation step is carried out by LC-MS. Also, the acid is preferably not hydrochloric acid ($pKa_1$ = -3.7) and/or sulfuric acid.

In other words, preferable examples of the acid anion include a methanesulfonate ion ($CH_3SO_3^-$), a nitrate ion ($NO_3^-$), a trifluoroacetate ion ($CF_3COO^-$), an oxalate ion ($C_2O_4^{2-}$), a hydrogen oxalate ion ($HC_2O_4^-$), a dihydrogen phosphate ion ($H_2PO_4^-$), a glycolate ion ($HOCH_2COO^-$), a formate ion ($HCOO^-$), an acetate ion ($CH_3COO^-$), and a hydrogen carbonate ion ($HCO_3^-$). Among these, the acid anion is more preferably one or more selected from the group consisting of a formate ion ($HCOO^-$), an oxalate ion ($C_2O_4^{2-}$), and an acetate ion ($CH_3COO^-$), and is even more preferably an oxalate ion ($C_2O_4^{2-}$), from the viewpoint of less noise in detection by a mass detector when the separation step is carried out by LC-MS. Also, the acid anion is preferably not a chloride ion ($Cl^-$) and/or a hydrogen sulfate ion ($HSO_4^-$).

In the present specification, the salt of a cation and an acid anion may be expressed as a salt of a base from which the cation is derived and an acid from which the acid anion is derived. For example, a salt formed from an ammonium ion and an oxalate ion may be expressed by $2NH_3 \cdot (COOH)_2$, a salt formed from a triethylammonium ion and a formate ion may be expressed by $Et_3N \cdot HCOOH$, a salt formed from a triethylammonium ion and a dihydrogen phosphate ion may be expressed by $Et_3N \cdot H_3PO_4$, and a salt formed from a trimethylammonium ion and a hydrochloric acid ion may be expressed by $Me_3N \cdot HCl$. When the "base from which the cation is derived" is an amine, the amine preferably does not correspond to the amine to be separated.

The concentration of the salt in the mobile phase (when two or more types of salts are contained in the mobile phase, the total concentration of the salts) is preferably set such that a value obtained by multiplying the concentration of the salt by the number of acid anions per one salt molecule (the value thereof corresponding to qY in the below-described Formula (1)) falls within a specific range. Specifically, the value obtained by multiplying the concentration of the salt by the number of acid anions per one salt molecule is usually set to 0.2 mM or more, preferably 0.4 mM or more, more preferably 1.0 mM or more, even more preferably 1.5 mM or more, and particularly preferably 2.0 mM or more, and is usually set to 50.0 mM or less, preferably 30.0 mM or less, more preferably 20.0 mM or less, and even more preferably 10.0 mM or less. That is, examples of preferable ranges of the salt concentration include ranges in which the value obtained by multiplying the salt concentration by the number of acid anions per one salt molecule is 0.2 mM or more and 30.0 mM or less, 0.4 mM or more and 50.0 mM or less, 1.0 mM or more and 30.0 mM or less, 1.5 mM or more and 20.0 mM or less, and 2.0 mM or more and 10.0 mM or less. When the concentration of the salt is within the above range, a sufficient amount of counter anions can be supplied into the mobile phase, and adverse effects on the chromatograph, the detector, and the like can be avoided.

2-3. Acid

**In** the present embodiment, when the mobile phase contains a salt of a cation and an acid anion, the requirement that the amine to be separated be retained in the stationary phase is satisfied, but it is also desirable that the amino group of the amine be protonated and present as an ammonium group. For this purpose, it is desirable that the acidity be adjusted by containing an acid in the mobile phase. The acid may be a single type, or may be a combination of any two or more types in any proportions.

Preferred examples of the acid for adjusting the acidity of the mobile phase include acids that release, through dissociation, protons and one or more anions selected from the acid anions given as examples of acid ions for forming the salt. That is, preferable examples of the acid for adjusting the acidity of the mobile phase include the acids from which the

acid anion is derived as described in the section "2-2. Salt of Cation and Acid Anion". The effect of improving the amine separation performance according to the present embodiment is considered to be based on the above-described principle, and therefore the acid for adjusting the acidity of the mobile phase may be the same as or different from the acid from which is derived the acid anion that forms the salt coexisting in the mobile phase. For example, the mobile phase may contain, as the salt, a salt of an ammonium ion and a hydrogen oxalate ion, as well as oxalic acid as an acid. **In** addition, for example, the mobile phase may contain, as the salt, a salt of an ammonium ion and a hydrogen oxalate ion, as well as formic acid as an acid. Furthermore, the acid for adjusting the acidity of the mobile phase is also preferably an acid having a weaker acidity than the acid from which is derived the acid anion that forms the salt.

In a case where the acidity of the acid for adjusting the acidity of the mobile phase is higher than the acidity of the acid from which is derived the acid anion that forms the salt, it is considered that proton exchange occurs, and the anion released from the acid for adjusting the acidity and the acid anion forming the salt are exchanged with each other. In this case, the anion released from the acid for adjusting the acidity of the mobile phase plays the same role as the acid anion forming the salt.

The acid for adjusting the acidity of the mobile phase is basically the same as the acid from which the acid anion is derived, which is described in the section "2-2. Salt of Cation and Acid Anion", but the following point should be noted regarding the acid dissociation constant of the acid from which the acid anion is derived.

Namely, the $pKa_n$ (n is the number of protons that are released) of the acid for adjusting the acidity of the mobile phase is preferably -2.9 or more, more preferably -2.0 or more, even more preferably -1.0 or more, particularly preferably 0.2 or more, and most preferably 1.0 or more, and is preferably 6.5 or less, more preferably 6.0 or less, even more preferably 5.5 or less, particularly preferably 4.5 or less, and most preferably 3.5 or less. That is, preferred ranges of the $pKa_n$ of the acid from which the acid anion is derived are, for example, -2.9 or more and 6.5 or less, -2.0 or more and 6.5 or less, -1.0 or more and 6.0 or less, 0.2 or more and 5.5 or less, and 1.0 or more and 4.5 or less. Alternatively, preferred ranges of the $pKa_n$ of the acid from which the acid anion is derived are, for example, -2.9 or more and 6.5 or less, -2.0 or more and 6.0 or less, -1.0 or more and 5.5 or less, 0.2 or more and 4.5 or less, and 1.0 or more and 3.5 or less.

When the mobile phase contains an acid having a $pKa_n$ equal to or more than the above lower limit together with the salt, the effect of improving the amine separation performance is further improved. In the case where the $pKa_n$ of the acid contained in the mobile phase together with the salt is equal to or less than the upper limit described above, when the separation target is an amino acid, the amino acid can be almost entirely protonated.

The upper limit of n is the maximum integer when the $pKa_n$ does not exceed the upper limit described above. The upper limit of n varies depending on the type of acid, but is usually an integer of 6 or less, preferably 4 or less, and more preferably 2 or less. Hereinafter, a case where the upper limit of the $pKa_n$ is 6.5 will be described with reference to specific examples. For phosphoric acid ($H_3PO_4$), $pKa_1$ = 2.12, $pKa_2$ = 7.21, and $pKa_3$ = 12.67, and therefore the upper limit of n is 1. For oxalic acid ($(COOH)_2$), $pKa_1$ = 1.27 and $pKa_2$ = 4.27, and therefore the upper limit of n is 2.

Preferable examples of the acid having a $pKa_n$ as described above include methanesulfonic acid ($pKa_1$ = -2.6), nitric acid ($pKa_1$ = -1.4), trifluoroacetic acid ($pKa_1$ = 0.23), oxalic acid ($pKa_1$ = 1.27, $pKa_2$ = 4.27), phosphoric acid ($pKa_1$ = 2.12), glycolic acid ($pKa_1$ = 3.1), formic acid ($pKa_1$ = 3.75), acetic acid ($pKa_1$ = 4.76), and carbonic acid ($pKa_1$ = 3.60).

When the mobile phase contains an acid, the concentration of the acid (when two or more types of acids are contained in the mobile phase, the total concentration of the acids) is usually more than 0 mM, preferably 0.5 mM or more, more preferably 1.0 mM or more, even more preferably 2.0 mM or more, and particularly preferably 5.0 mM or more, and is preferably 50.0 mM or less, more preferably 40.0 mM or less, even more preferably 30.0 mM or less, and particularly preferably 20.0 mM or less. That is, examples of preferable ranges of the acid concentration in the mobile phase include a range of more than 0 mM and 50.0 mM or less, a range of 0.5 mM or more and 50.0 mM or less, a range of 1.0 mM or more and 40.0 mM or less, a range of 2.0 mM or more and 30.0 mM or less, and a range of 5.0 mM or more and 20.0 mM or less. When the concentration of the acid is within the above range, it is possible to prevent an excessive amount of the acid from adversely affecting the chromatograph, the detector, and the like while ensuring appropriate retention of the amine.

2-4. Quantitative Relationship Between Salt and Acid in Mobile Phase

In the mobile phase used in the present embodiment, the salt and the acid, which is an optional component, are preferably in a specific quantitative relationship. The quantitative relationship between these components can be expressed by the following Formula (1). The following Formula (1) presents the relationship between the concentration of the acid and the concentration of the salt. In other words, it can be said that Formula (1) represents the degree of neutralization of the acid in a case where it is assumed that the mobile phase is prepared by neutralizing the acid with a base.

In the mobile phase, the value calculated by the following Formula (1) is preferably 0.01 or more, more preferably 0.05 or more, still more preferably 0.10 or more, particularly preferably 0.20 or more, and most preferably 0.30 or more, and is preferably 0.90 or less, more preferably 0.80 or less, still more preferably 0.70 or less, and particularly preferably 0.60 or less. That is, examples of preferable ranges of the value calculated by Formula (1) include a range of 0.01 or more and 0.90

or less, a range of 0.05 or more and 0.80 or less, a range of 0.10 or more and 0.70 or less, a range of 0.20 or more and 0.60 or less, and a range of 0.30 or more and 0.60 or less. When the value calculated by Formula (1) is within the above range, a sufficient amount of counter anions can be supplied into the mobile phase, and the amino group of the amine to be separated can be efficiently converted into an ammonium group by the proton generated from the acid. The case where the value calculated by Formula (1) is 1 means that the mobile phase does not contain an acid.
[Math. 2]

$$\frac{qY}{X + qY} \qquad (1)$$

X is the concentration (mM) of the acid in the mobile phase and is as described above.
Y is the concentration (mM) of the salt in the mobile phase and is as described above.

Moreover, q is the number of acid anions per salt molecule. More specifically, a salt ($2NH_3 \cdot (COOH)_2$) of an ammonium ion and an oxalate ion has a q value of 1. A salt ($Et_3N \cdot CF_3COOH$) of a triethylammonium ion and a trifluoroacetate ion has a q value of 1. Moreover, a salt (TMEDA·2HCOOH) of an ammonium ion of tetramethylethylenediamine and a formate ion has a q value of 2.

2-5. Method for Preparing Mobile Phase

The mobile phase can be prepared by a known solution preparation method or a method equivalent to a known solution preparation method.
Known methods include a method for forming a salt from an acid and a base and using the salt to prepare the mobile phase. For example, when one or more salts selected from the group consisting of tetrahydroammonium salts, primary ammonium salts, secondary ammonium salts, and tertiary ammonium salts are used as the salt, as described below in Examples, a mobile phase containing a desired component can be easily prepared by causing contact between an acid corresponding to a target acid anion and a base corresponding to a target cation (e.g., an amine and aqueous ammonia) in a solvent of the mobile phase to convert some or all of the acid into a salt, and then increasing the volume to a predetermined volume with the same solvent in a measuring flask. This technique of preparing a mobile phase by bringing an acid and a base into contact with each other in a solvent in this manner rather than directly bringing the acid and the base into contact with each other is suitable in that the technique is simple, and problems caused by the heat of neutralization such as vaporization of some of the components can be avoided.
When a salt of a cation and an acid anion is commercially available or can be easily procured, the mobile phase can be prepared by dissolving the commercially available product in a solvent and, as necessary, mixing with an acid for adjusting the acidity.

3. Stationary Phase

The stationary phase of the present embodiment is a separating agent in which a ligand having a crown ether-like cyclic structure is supported on a carrier.
In the present specification, the term ligand means a compound that is supported on a carrier and exhibits physical affinity for the separation target and, as necessary, a chiral recognition ability. That is, the ligand having a crown ether-like cyclic structure is a compound in which a polyoxyethylene chain represented by Formula (I) is chemically bonded to an aliphatic, alicyclic, or aromatic hydrocarbon to form a macrocyclic structure.

$$*\text{-}O(CH_2CH_2O)_n\text{-}* \qquad (I)$$

In the formula, n can be selected, as appropriate, from integers of 4 to 6 depending on the hydrocarbon to which the amino group of the amine and the crown ether backbone are bonded. For example, a ligand represented by Formula (II) or (III) described below is suitably used for separation of a primary amine because n is 5, and thus the ligand has a crown ether-like cyclic structure of a size suitable for inclusion of a primary ammonium group. The hydrogen atom of the ethylene group in the repeating unit may be substituted by any functional group, but is preferably unsubstituted.
In the present embodiment, when enantiomers are to be separated, a compound in which the crown ether-like cyclic structure is bonded to a homochiral structure is used as the ligand. Examples of such ligands include ligands represented by Formula (II) described in JP H2-69472 A and WO 2012/050124, and ligands represented by Formula (III) described in JP 2014-169259 A. In addition, a ligand for which the phenyl groups at the 3 and 3' positions of the 1,1'-binaphthyl structure in Formula (II) are substituted with a halogen atom such as a bromine atom, an alkyl group such as a methyl group, a substituted aromatic group, a heterocyclic group, or the like (Peng Wu, et. al., Chinese Journal of Chemistry, 2017, 35, pp. 1037-1042) may also be used, but the ligand is not limited to these types of ligands. Moreover, ligands that are effective in separating enantiomers are often also effective in separating molecules that are similar to each other.

[Chem. 1]

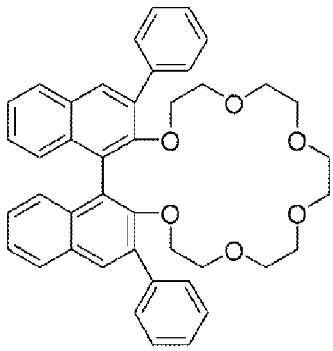

(II)

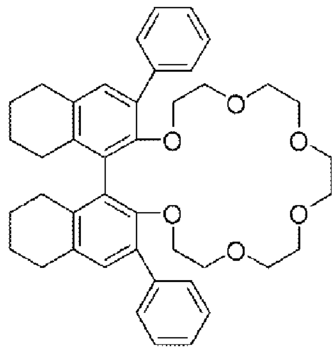

(III)

The ligand is used as the stationary phase in a state of being supported by a carrier. A known method may be adopted as the method of supporting the ligand on a carrier, and for example, a method in which the ligand is supported on the carrier by chemical bonding, such as covalent bonding, can be suitably adopted. A specific example thereof is a method in which a reactive group is introduced into the ligand, the ligand raw material, or a ligand intermediate, and this substituent is reacted with a reactive group present on the carrier surface. The reactive group present on the surface of the carrier may be a group present on the surface of the untreated carrier, or may be a group introduced to the surface of the carrier by subjecting the carrier to a surface treatment using a surface treatment agent, for example, a silane coupling agent such as 3-aminopropyltriethoxysilane or 3-glycidyloxypropyltrimethoxysilane. Another method for supporting the ligand on a carrier is a method in which a pair of so-called click reaction partners are bonded to the stationary phase and a ligand, respectively, and reacted with each other. Alternatively, an insolubilized layer can be formed on the surface of the carrier not only by a method of forming a bond between the ligand and the carrier, but also by forming a so-called crosslinking bond between atomic groups including the ligand. Furthermore, the ligand may be supported on the carrier by another known supporting method, for example, a method of physically adsorbing (coating) the ligand on the carrier.

The carrier is not particularly limited as long as the ligand can be fixed to the carrier. The carrier may be an inorganic carrier or an organic carrier, but is preferably an inorganic carrier. Examples of the inorganic carrier include silica gel, alumina, magnesia, glass, kaolin, titanium oxide, silicate, zirconia, and hydroxyapatite. Examples of the organic carrier include crosslinked polystyrene, crosslinked poly(meth)acrylamide, crosslinked poly(meth)acrylate, and polysaccharides. Such an organic carrier is preferably insolubilized by crosslinking using a crosslinking agent.

The form of the carrier is not particularly limited, and examples thereof include particles and a porous cylindrical body (monolith) that is housed in a column tube with a liquid tight seal. Another example of the carrier is an inner wall of a capillary. When the separation factor is sufficiently large, a membrane-type carrier may be used and developed in a thickness direction, or may be developed in a plane direction as thin-layer chromatography.

In the present embodiment, from the perspective of improving separation performance, the carrier is preferably a porous body, and is more preferably a porous body having a BET specific surface area of 100 $m^2/g$ or more and 600 $m^2/g$ or less. From the perspective of improving separation performance, the porous body preferably has a pore diameter of from 60 Å to 300 Å, as measured by mercury intrusion porosimetry.

In the present embodiment, the carrier is preferably a silica gel. This is because silica gel excels in the property described above, namely, separation performance, and is also hard and strong. The silica gel to be used may be a fully porous silica gel, a so-called core-shell type silica gel, or a silica gel having a chemically modified surface.

4. Chromatography

The chromatography used in the present embodiment is not particularly limited, and examples thereof include liquid chromatography and supercritical fluid chromatography. The chromatography can be carried out using a commercially available chromatograph such as a liquid chromatograph or a supercritical fluid chromatograph. The various conditions such as the column equilibration conditions and flow rate can be appropriately determined depending on, for

example, the column size, the sample volume, and the type of mobile phase.

In the present embodiment, when liquid chromatography is adopted as the chromatography method, amine separation and mass spectrometry of the separated amine may be sequentially performed by liquid chromatography-mass spectrometry (LC-MS) in which liquid chromatography is combined with mass spectrometry (MS). The mass spectrometry of the amine may be quantitative analysis or qualitative analysis of the separated amine.

A known mass spectrometry method used in LC-MS can be employed for the mass spectrometry. Examples of ionization methods in mass spectrometry include atmospheric pressure chemical ionization (APCI), atmospheric pressure photoionization (APPI), electrospray ionization (ESI), fast atom bombardment (FAB), and thermospray ionization(TSP), and the ionization method can be appropriately selected depending on, for example, the type of salt, the type of acid, the type of amine, and the purpose of analysis. The mass detector to be used can be appropriately selected from a quadrupole mass spectrometer (Q-MS), an ion trap mass spectrometer (IT-MS), a time-of-flight mass spectrometer (TOF-MS), and the like depending on, for example, the required sensitivity and resolution.

## EXAMPLES

Hereinafter, the present disclosure will be described more specifically with reference to Examples, but the present disclosure is not limited to the following Examples as long as there is not deviation from the gist of the present disclosure.

### [Preparation of Mobile Phase]

Next will be described a method for preparing a mobile phase C containing oxalic acid at a concentration of 7.5 mM, triethylamine oxalate ($2Et_3N\cdot(COOH)_2$) at a concentration of 2.5 mM, and a solvent, i.e., a water/acetonitrile (5/95 (v/v)) mixed solvent. Other mobile phases were prepared by the method for preparing the mobile phase C or a method similar thereto with the salts, acids and solvents as described in Table 2.

An amount of 316.9 mg of oxalic acid dihydrate was weighed and transferred to a 250 mL volumetric flask. Approximately 50 mL of a water/acetonitrile (5/95 (v/v)) mixed solvent was added to the volumetric flask, and then 1336.4 mg of acetonitrile and 127.3 mg of triethylamine were added thereto. Subsequently, the mixture was diluted in the volumetric flask by the further addition of the water/acetonitrile (5/95 (v/v)) mixed solvent, and the mixture was stirred until no undissolved matter remained, whereby the mobile phase C was prepared.

When an aqueous acid solution was used in the preparation of the mobile phase, firstly, a predetermined amount calculated from the concentration described on the warranty certificate of the manufacturer of the aqueous acid solution was weighed out. Next, acetonitrile was added to the aqueous acid solution to prepare a mixed solution. In this case, acetonitrile was added in such an amount that the volume ratio of water/acetonitrile in the mixed solution was the same as the volume ratio of water/acetonitrile of the target solvent. Subsequently, the water/acetonitrile mixed solvent mixed at the above-mentioned volume ratio was added to the mixed solution to increase the volume.

The specific gravity of water was 1.00 g/mL, and the specific gravity of acetonitrile was 0.783 g/mL.

### [Amine Separation]

Next, amine separation was carried out using a column ("CROWNPAK CR-I (-)", available from Daicel Corporation; inner diameter of 3 mm; length of 150 mm) packed with a chiral stationary phase in which a ligand having a crown ether-like cyclic structure represented by Formula (IV) was supported by silica gel via a chemical bond, and using, as a liquid chromatograph, a high performance liquid chromatograph ("LC-20AD", available from Shimadzu Corporation). In this case, the amine to be separated was dissolved in a water/acetonitrile (1/1 (v/v)) mixed solvent to a concentration of about 0.1% w/v, and 2 μL of the resulting solution was injected into the column using an autosampler. The mobile phase was fed at a rate of 0.43 mL/min to the column adjusted to a temperature of 30°C.

A photodiode array detector ("SPD-M20A" available from Shimadzu Corporation, detection wavelength of 254 nm) was used as a detector, and data obtained by the detector were analyzed using data analysis software ("LC Solution" available from Shimadzu Corporation).

[Chem. 2]

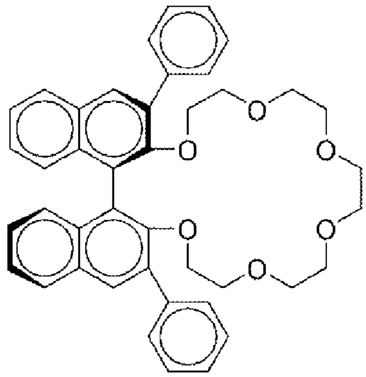

(IV)

[Examples 1 to 9 and Comparative Examples 1 to 8]

Chiral separation of dl-1-phenylethylamine was carried out in accordance with the method described in the "Amine Separation" section above using the mobile phases shown in Table 2. The results are illustrated in FIGS. 1 to 17.

[Examples 10 to 12 and Comparative Examples 9 and 10]

Chiral separation of dl-tryptophan was carried out in accordance with the method described in the "Amine Separation" section above using the mobile phases shown in Table 2. The results are illustrated in FIGS. 18 to 22.

[Example 13]

Chiral separation of dl-tryptophan was carried out in the same manner as in Example 1, except that the mobile phase shown in Table 2 was used, the following LC-MS device was used in the above-described "Amine Separation", and the column temperature was set to 20°C. The results are illustrated in FIG. 23.

LC-MS Device

· Liquid Chromatograph
Device: "Waters ACQUITY UPLC H-Class PLUS" available from Waters Corporation
· Mass Detector

Device: "Waters ACQUITY QDa" available from Waters Corporation
Ionization method: ESI
Measurement mode: Positive
Detection MS: m/z205
Probe temperature: 400°C
Source temperature: 120°C

In Example 1, a mobile phase containing nitric acid and triethylamine nitrate was used, and in Comparative Example 1, a mobile phase containing nitric acid but not containing the salt was used. When the differences between the retention times of the peaks of d-1-phenylethylamine and l-1-phenylethylamine were compared in Example 1 and Comparative Example 1, the difference was about 3.5 minutes in Example 1, while the difference was a little less than 2 minutes in Comparative Example 1.

When the differences between the retention times of the two peaks were compared in Example 2, in which a mobile phase containing trifluoroacetic acid and triethylamine trifluoroacetate was used, and Comparative Examples 2 and 3, in which a mobile phase containing trifluoroacetic acid but not containing the salt was used, the difference in the retention times of the two peaks was about 3 minutes in Example 2, while the difference thereof was about 1 minute in Comparative Examples 2 and 3. In Comparative Example 3, although the concentration of the trifluoroacetic acid was 10 times that in Example 2 and Comparative Example 2, the separation performance was hardly improved.

In Example 3, in which a mobile phase containing oxalic acid and triethylamine hydrogen oxalate was used, the

difference in retention times of the two peaks was about 7 minutes, and very high separation performance was exhibited. In contrast, in Comparative Example 4, in which a mobile phase containing oxalic acid but not containing the salt was used, dl-1-phenylethylamine failed to be separated into individual enantiomers. In Examples 4 and 5, in which the type of salt in the mobile phase differed from that in Example 3, the two peaks were sufficiently separated from each other. The results indicate that the amines were efficiently separated.

Regarding the mobile phase containing oxalic acid, the same trends as described above were observed even when the separation target was changed to dl-tryptophan. Specifically, in Comparative Example 9, in which a mobile phase not containing a salt of a cation and an acid anion was used, the difference between the retention times of the two peaks was small, i.e., about 0.7 minutes, whereas in Examples 10 to 12, in which a mobile phase containing dimethylamine or a hydrogen oxalate of ammonia was used, the difference between the retention times of the two peaks was from approximately 1.5 minutes to approximately 12 minutes.

In comparison of Example 3, in which a mobile phase containing a water/acetonitrile (5/95 (v/v)) mixed solvent was used, and Comparative Example 8, in which a water/acetonitrile (95/5 (v/v)) mixed solvent was contained, dl-1-phenylethylamine failed to be separated into individual enantiomers in Comparative Example 8, whereas very high separation performance was exhibited in Example 3. Also in Comparative Example 10, in which the same mobile phase as in Comparative Example 8 was used, dl-tryptophan failed to be separated into individual enantiomers. These results indicate that the effect of improving amine separation performance by containing, in the mobile phase, a salt of a cation and an acid anion is remarkably exhibited in a mobile phase having a low water content.

When the differences between the retention times of the two peaks were compared in Example 6, in which a mobile phase containing phosphoric acid and triethylamine dihydrogen phosphate was used, and Comparative Example 5, in which a mobile phase containing phosphoric acid but not the salt thereof was used, the difference was approximately 4.5 minutes in Example 6, whereas the difference thereof was approximately 1 minute in Comparative Example 5.

**In** Examples 7 and 8, in which a mobile phase containing formic acid and triethylamine formate was used, the difference in the retention times between the two peaks was about 16 minutes and about 5 minutes, respectively. Thus, very high separation performance was exhibited in these Examples. In contrast, in Comparative Example 6, in which a mobile phase containing formic acid but not containing the salt thereof was used, dl-1-phenylethylamine failed to be separated into individual enantiomers.

When the differences between the retention times of the two peaks were compared in Example 9, in which a mobile phase containing methanesulfonic acid and triethylamine methanesulfonate was used, and Comparative Example 7, in which a mobile phase containing methanesulfonic acid but not containing the salt thereof was used, the difference was approximately 2 minutes in Example 9, whereas the difference thereof was approximately 1.5 minutes in Comparative Example 7.

In addition, the results of Example 13 showed that even when chromatography was carried out by LC-MS, there were few unnecessary peaks in the liquid chromatogram, and there was minimal noise in the detection by the mass detector. These results indicate that the present separation method can also be applied to LC-MS.

[Table 2]

Table 2

| | Mobile Phase | Salt | | | | Acid | | Formula (1) calculated Value | Solvent | Liquid chromatogram |
|---|---|---|---|---|---|---|---|---|---|---|
| | | Salt | pKa* | q | Concentration Y (mM) | Acid | Concentration X (mM) | | | |
| Example 1 | Mobile phase A | $Et_3N \cdot HNO_3$ | -1.4 | 1 | 5.0 | $HNO_3$ | 5.0 | 0.50 | $H_2O/CH_3CN$ (5/95 (v/v)) | FIG. 1 |
| Example 2 | Mobile phase B | $Et_3N \cdot CF_3COOH$ | 0.23 | 1 | 5.0 | $CF_3COOH$ | 5.0 | 0.50 | $H_2O/CH_3CN$ (5/95 (v/v)) | FIG. 2 |
| Example 3 | Mobile phase C | $Et_3N \cdot (COOH)_2$ | 1.27 | 1 | 5.0 | $(COOH)_2$ | 5.0 | 0.50 | $H_2O/CH_3CN$ (5/95 (v/v)) | FIG. 3 |
| Example 4 | Mobile phase D | $NH_3 \cdot (COOH)_2$ | 1.27 | 1 | 0.8 | $(COOH)_2$ | 9.2 | 0.08 | $H_2O/CH_3CN$ (5/95 (v/v)) | FIG. 4 |
| Example 5 | Mobile phase E | $NHMe_2 \cdot (COOH)_2$ | 1.27 | 1 | 2.5 | $(COOH)_2$ | 7.5 | 0.25 | $H_2O/CH_3CN$ (5/95 (v/v)) | FIG. 5 |
| Example 6 | Mobile phase F | $Et_3N \cdot H_3PO_4$ | 2.12 | 1 | 1.75 | $H_3PO_4$ | 3.35 | 0.343 | $H_2O/CH_3CN$ (10/90 (v/v)) | FIG. 6 |
| Example 7 | Mobile phase G | $Et_3N \cdot HCOOH$ | 3.75 | 1 | 1.10 | HCOOH | 8.85 | 0.111 | $H_2O/CH_3CN$ (5/95 (v/v)) | FIG. 7 |
| Example 8 | Mobile phase H | $Et_3N \cdot HCOOH$ | 3.75 | 1 | 1.16 | HCOOH | 8.76 | 0.117 | $H_2O/CH_3CN$ (7.5192.5 (v/v)) | FIG. 8 |
| Comparative Example 1 | Mobile phase a | - | - | - | 0 | $HNO_3$ | 5.0 | 0 | $H_2O/CH_3CN$ (5/95 (v/v)) | FIG. 9 |
| Comparative Example 2 | Mobile phase b | - | - | - | 0 | $CF_3COOH$ | 5.0 | 0 | $H_2O/CH_3CN$ (5/95 (v/v)) | FIG. 10 |
| Comparative Example 3 | Mobile phase c | - | - | - | 0 | $CF_3COOH$ | 50.0 | 0 | $H_2O/CH_3CN$ (5/95 (v/v)) | FIG. 11 |
| Comparative Example 4 | Mobile phase d | - | - | - | 0 | $(COOH)_2$ | 10.0 | 0 | $H_2O/CH_3CN$ (5/95 (v/v)) | FIG. 12 |
| Comparative Example 5 | Mobile phase e | - | - | - | 0 | $H_3PO_4$ | 5.0 | 0 | $H_2O/CH_3CN$ (5/95 (v/v)) | FIG. 13 |

(continued)

| | Mobile Phase | Salt | | | | Acid | | Formula (1) calculated Value | Solvent | Liquid chromatogram |
|---|---|---|---|---|---|---|---|---|---|---|
| | | Salt | pKa* | q | Concentration Y (mM) | Acid | Concentration X (mM) | | | |
| Comparative Example 6 | Mobile phase f | - | - | - | 0 | HCOOH | 5.0 | 0 | $H_2O/CH_3CN$ (5/95 (v/v)) | FIG. 14 |
| Example 9 | Mobile Phase I | $Et_3N \cdot CH_3SO_3H$ | -2.6 | 1 | 2.5 | $CH_3SO_3H$ | 2.5 | 0.50 | $H_2O/CH_3CN$ (7.5/92.5 (v/v)) | FIG. 15 |
| Comparative Example 7 | Mobile phase g | - | - | - | 0 | $CH_3SO_3H$ | 5.0 | 0 | $H_2O/CH_3CN$ (7.5192.5 (v/v)) | FIG. 16 |
| Comparative Example 8 | Mobile phase h | $Et_3N \cdot (COOH)_2$ | 1.27 | 1 | 5.0 | $(COOH)_2$ | 5.0 | 0.50 | $H_2O/CH_3CN$ (95/5 (v/v)) | FIG. 17 |
| Example 10 | Mobile phase E | $NHMe_2 \cdot (COOH)_2$ | 1.27 | 1 | 2.5 | $(COOH)_2$ | 7.5 | 0.25 | $H_2O/CH_3CN$ (5/95 (v/v)) | FIG. 18 |
| Example 11 | Mobile phase D | $NH_3 \cdot (COOH)_2$ | 1.27 | 1 | 0.8 | $(COOH)_2$ | 9.2 | 0.08 | $H_2O/CH_3CN$ (5/95 (v/v)) | FIG. 19 |
| Example 12 | Mobile phase J | $NH_3 \cdot (COOH)_2$ | 1.27 | 1 | 2.5 | $(COOH)_2$ | 7.5 | 0.25 | $H_2O/CH_3CN$ (10/90 (v/v)) | FIG. 20 |
| Comparative Example 9 | Mobile phase d | - | - | - | 0 | $(COOH)_2$ | 10.0 | 0 | $H_2O/CH_3CN$ (5/95 (v/v)) | FIG. 21 |
| Comparative Example 10 | Mobile phase h | $Et_3N \cdot (COOH)_2$ | 1.27 | 1 | 5.0 | $(COOH)_2$ | 5.0 | 0.50 | $H_2O/CH_3CN$ (95/5 (v/v)) | FIG. 22 |
| Example 13 | Mobile phase K | $NHMe_2 \cdot (COOH)_2$ | 1.27 | 1 | 2.3 | $(COOH)_2$ | 7.7 | 0.23 | $H_2O/CH_3CN$ (5/95 (v/v)) | FIG. 23 |

* $pKa_n$ of an acid from which an acid anion is derived, the acid anion being an anion component of a salt

[Examples 14 to 16]

Chiral separations of Phe-Gly-NH2 (Example 14), dl-tryptophan (Example 15), and dl-1-phenylethylamine (Example 16) were carried out in accordance with the method described in the [Amine Separation] section above using a mobile phase containing oxalic acid and triethylamine hydrogen oxalate. FIG. 24 illustrates a graph in which the retention times of the two peaks are plotted against the concentrations of oxalic acid and triethylamine hydrogen oxalate in the mobile phase.

The results of Examples 14 to 16 indicate that when the value calculated by Formula (1) for the mobile phase is within the specific range described above, the amine separation performance is improved.

INDUSTRIAL APPLICABILITY

High amine separation performance can be realized through the amine separation method according to at least several embodiments of the present disclosure, even when a mobile phase having a low water content is used. It is considered that the amine separation method of the present disclosure can be applied to LC-MS by appropriately selecting the components contained in the mobile phase from among components which can avoid or reduce adverse effects on detection using a mass spectrometer.

Accordingly, the amine separation method can be widely applied to analysis and purification using various liquid chromatography methods, and an expansion of this method in various fields such as organic chemistry, medicine, and pharmacology is anticipated.

**Claims**

1. An amine separation method comprising separating an amine through chromatography using:

   a stationary phase comprising a ligand having a crown ether-like cyclic structure and being supported on a carrier; and
   a mobile phase comprising a salt of a cation and an acid anion at a concentration of 0.2 mM or more and 50.0 mM or less and comprising a solvent having a water content of 50 vol.% or less.

2. The amine separation method according to claim 1, wherein the mobile phase comprises an acid at a concentration of more than 0 mM and 50.0 mM or less.

3. The amine separation method according to claim 1 or 2, wherein the solvent has a water content of 20 vol.% or less.

4. The amine separation method according to any one of claims 1 to 3, wherein a liquid other than water in the solvent is one or more selected from the group consisting of an organic solvent and subcritical or supercritical carbon dioxide.

5. The amine separation method according to claim 4, wherein the organic solvent is one or more selected from the group consisting of acetonitrile, acetone, methanol, ethanol, 2-propanol, hexane, tetrahydrofuran, and tert-butyl methyl ether.

6. The amine separation method according to any one of claims 1 to 3, wherein the solvent comprises acetonitrile in an amount of 70 vol.% or more.

7. The amine separation method according to any one of claims 1 to 6, wherein an acid from which the acid anion is derived has a $pKa_n$ of -2.0 or more and 6.5 or less in water at 25°C:
   wherein, n is a valence of the acid anion.

8. The amine separation method according to any one of claims 1 to 6, wherein the acid anion is one or more selected from the group consisting of a nitrate ion, a trifluoroacetate ion, an oxalate ion, a hydrogen oxalate ion, a dihydrogen phosphate ion, a glycolate ion, a formate ion, an acetate ion, and a hydrogen carbonate ion.

9. The amine separation method according to any one of claims 1 to 6, wherein the acid anion is one or more selected from the group consisting of an oxalate ion, a formate ion, and an acetate ion.

10. The amine separation method according to any one of claims 1 to 9, wherein the cation is one or more selected from

the group consisting of a tetrahydroammonium ion, a secondary ammonium ion, and a tertiary ammonium ion.

**11.** The amine separation method according to claim 2, wherein a value calculated by Formula (1) for the mobile phase is 0.01 or more and 0.90 or less:
[Math. 1]

$$\frac{qY}{X + qY} \qquad (1)$$

wherein,

X is a concentration (mM) of the acid in the mobile phase;
Y is a concentration (mM) of the salt in the mobile phase; and
q is the number of acid anions per molecule of the salt.

**12.** The amine separation method according to any one of claims 1 to 11, wherein the chromatography is carried out by liquid chromatography-mass spectrometry.

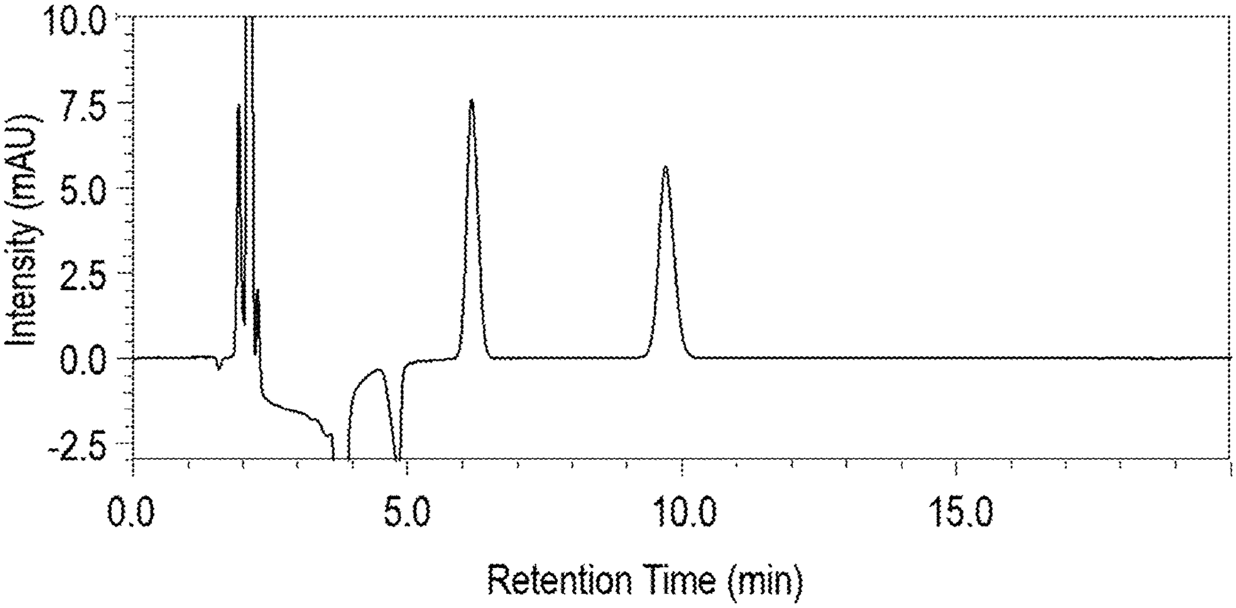
10.0
7.5
5.0
2.5
0.0
-2.5
Intensity (mAU)
0.0
5.0
10.0
15.0
Retention Time (min)

FIG. 1

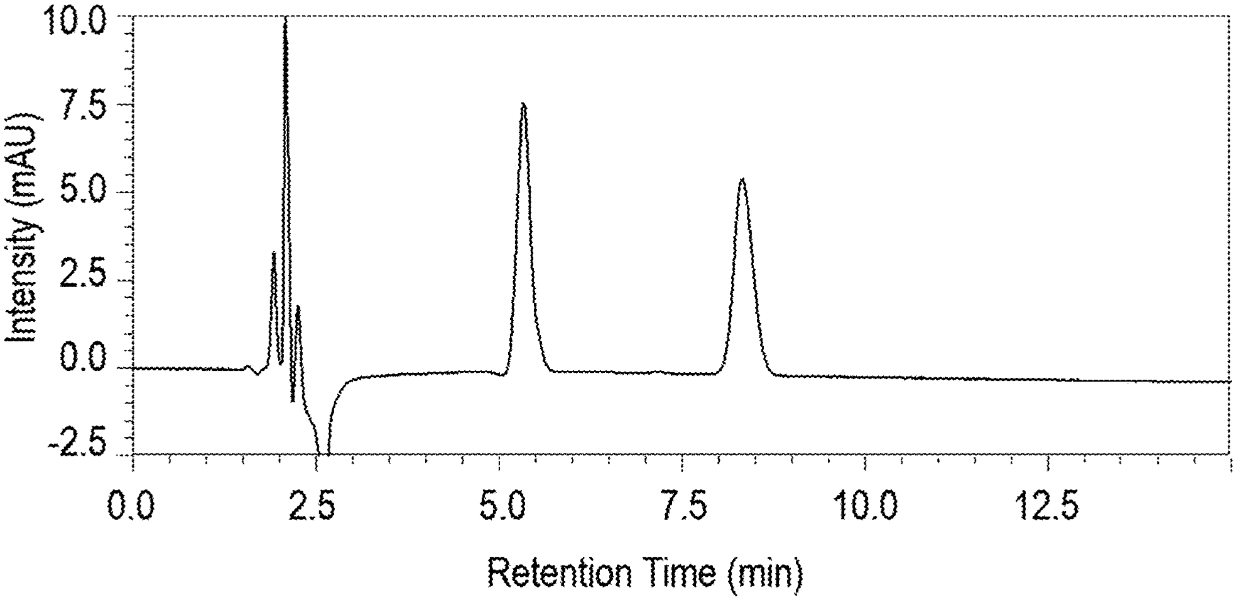
10.0
7.5
5.0
2.5
0.0
-2.5
Intensity (mAU)
0.0
2.5
5.0
7.5
10.0
12.5
Retention Time (min)

FIG. 2

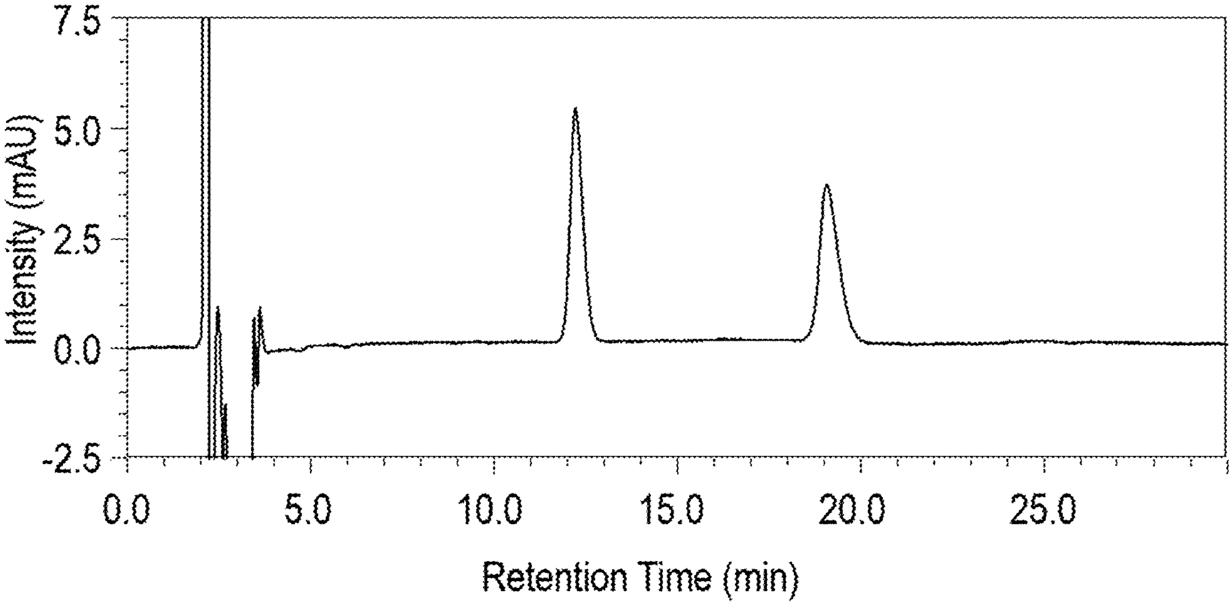
7.5
5.0
2.5
0.0
-2.5
Intensity (mAU)
0.0
5.0
10.0
15.0
20.0
25.0
Retention Time (min)

FIG. 3

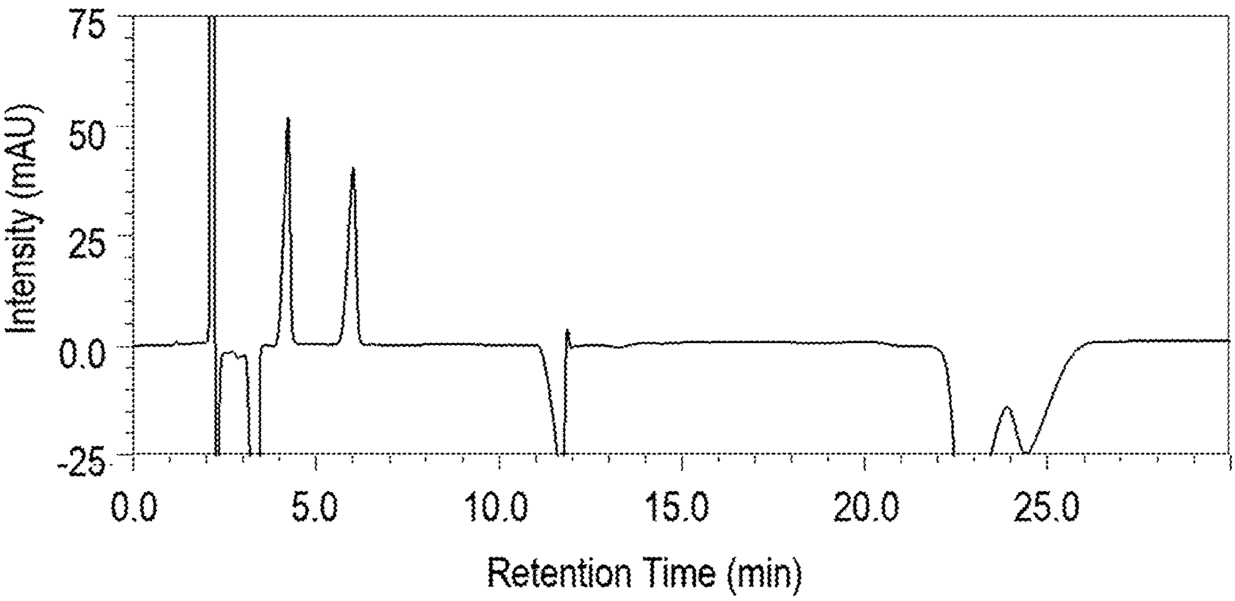
75
50
25
0.0
-25
Intensity (mAU)
0.0
5.0
10.0
15.0
20.0
25.0
Retention Time (min)

FIG. 4

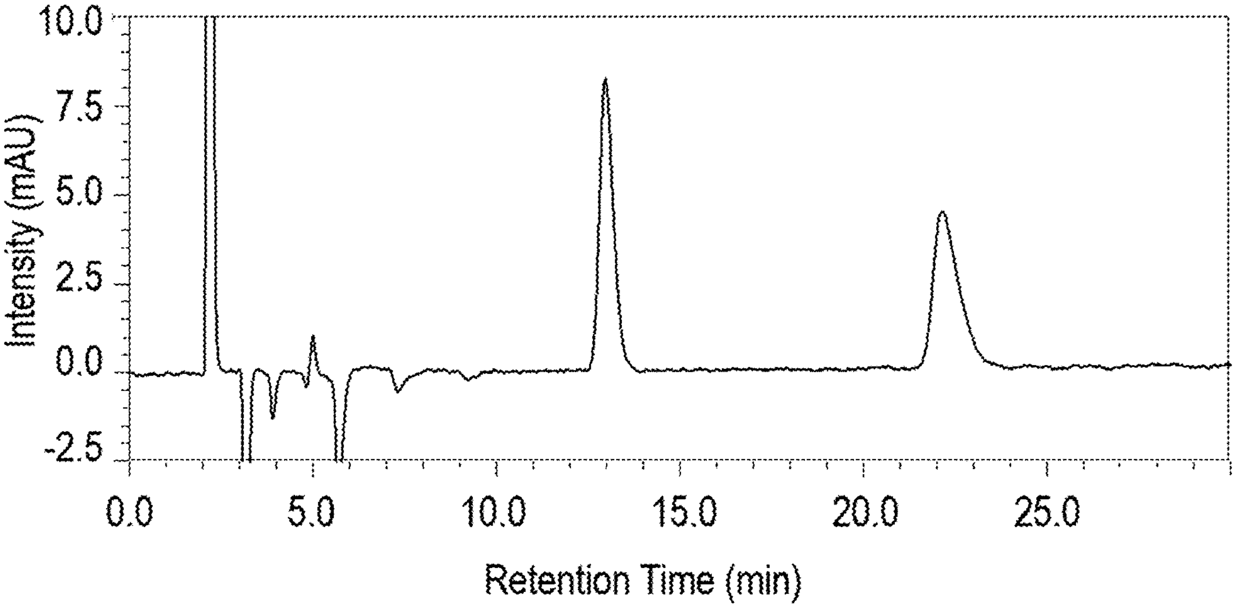
10.0
7.5
5.0
2.5
0.0
-2.5
Intensity (mAU)
0.0
5.0
10.0
15.0
20.0
25.0
Retention Time (min)

FIG. 5

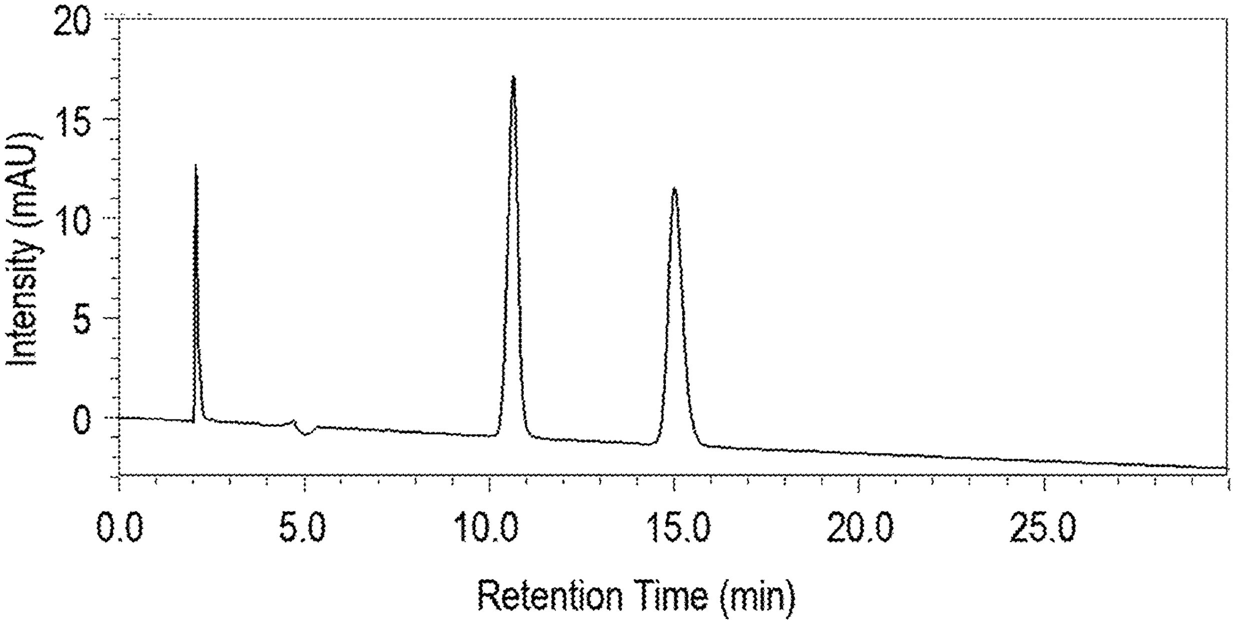
20
15
10
5
0
Intensity (mAU)
0.0
5.0
10.0
15.0
20.0
25.0
Retention Time (min)

FIG. 6

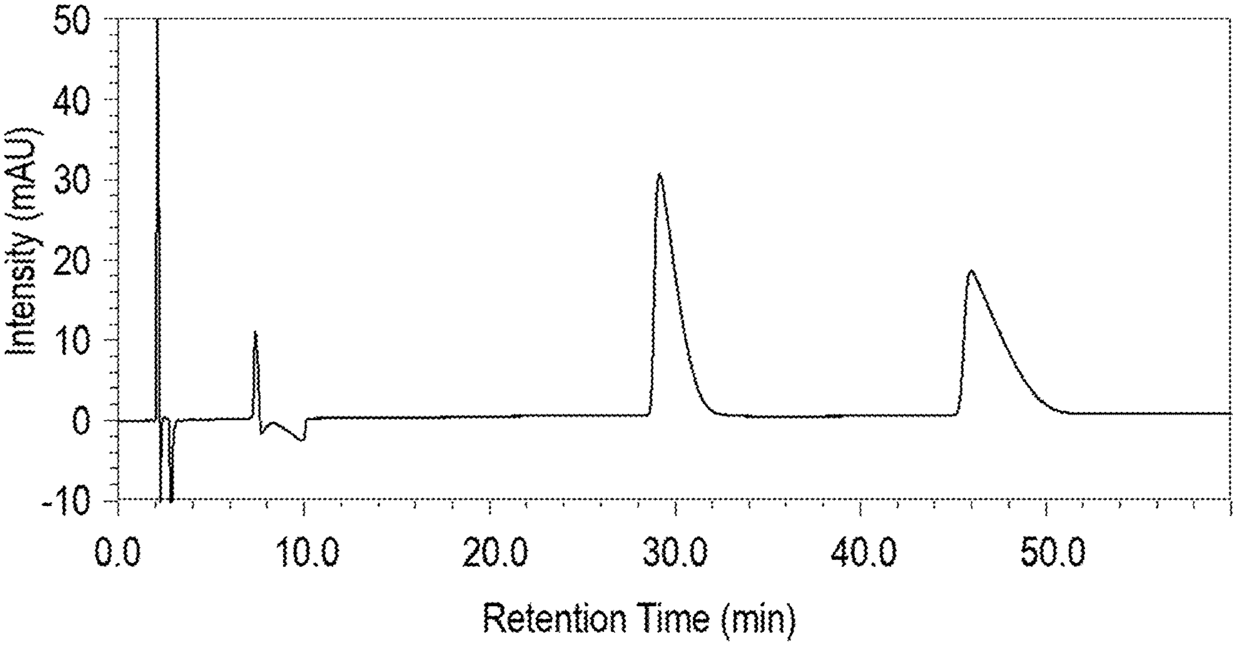
50
40
30
20
10
0
-10
Intensity (mAU)
0.0
10.0
20.0
30.0
40.0
50.0
Retention Time (min)

FIG. 7

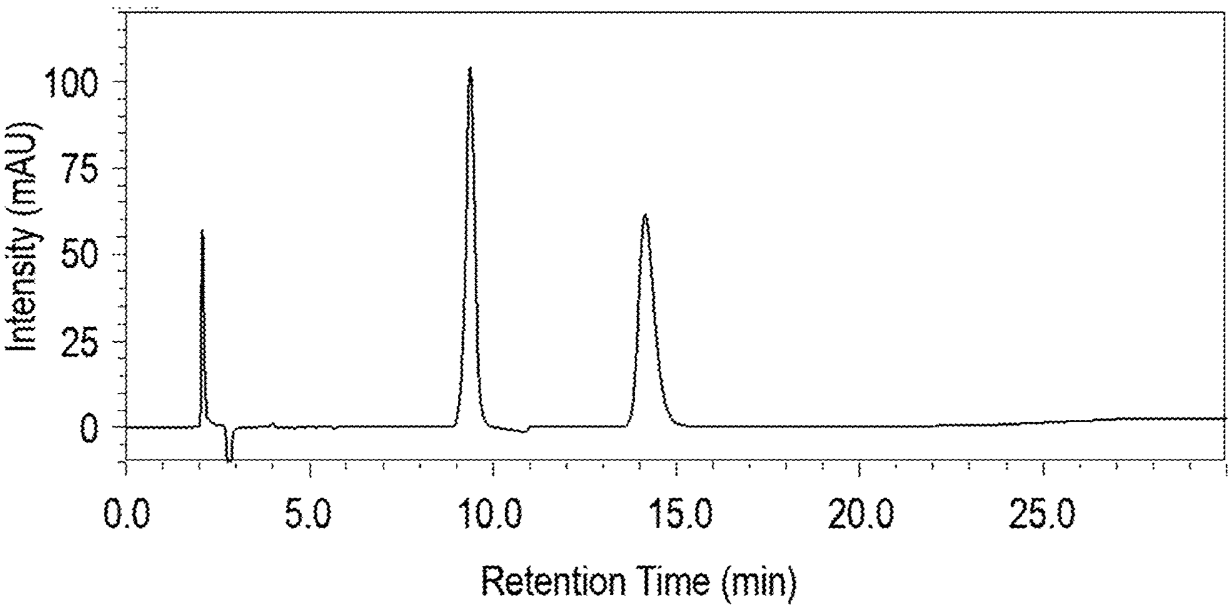
100
75
50
25
0
Intensity (mAU)
0.0
5.0
10.0
15.0
20.0
25.0
Retention Time (min)

FIG. 8

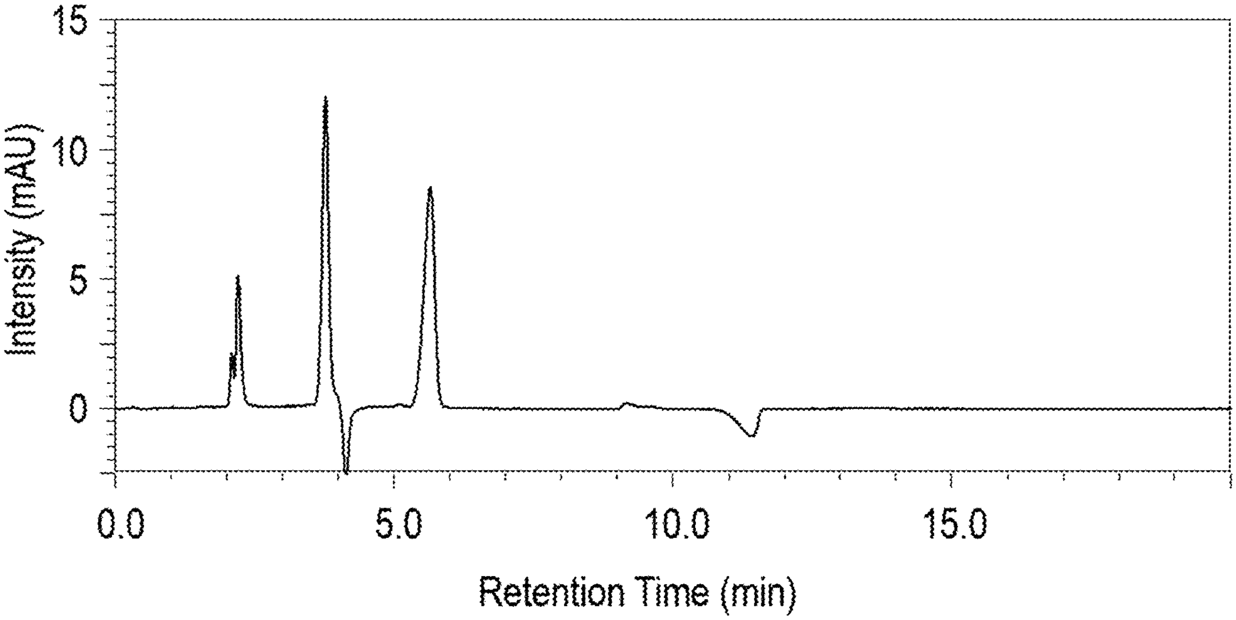
15
10
5
0
Intensity (mAU)
0.0
5.0
10.0
15.0
Retention Time (min)

FIG. 9

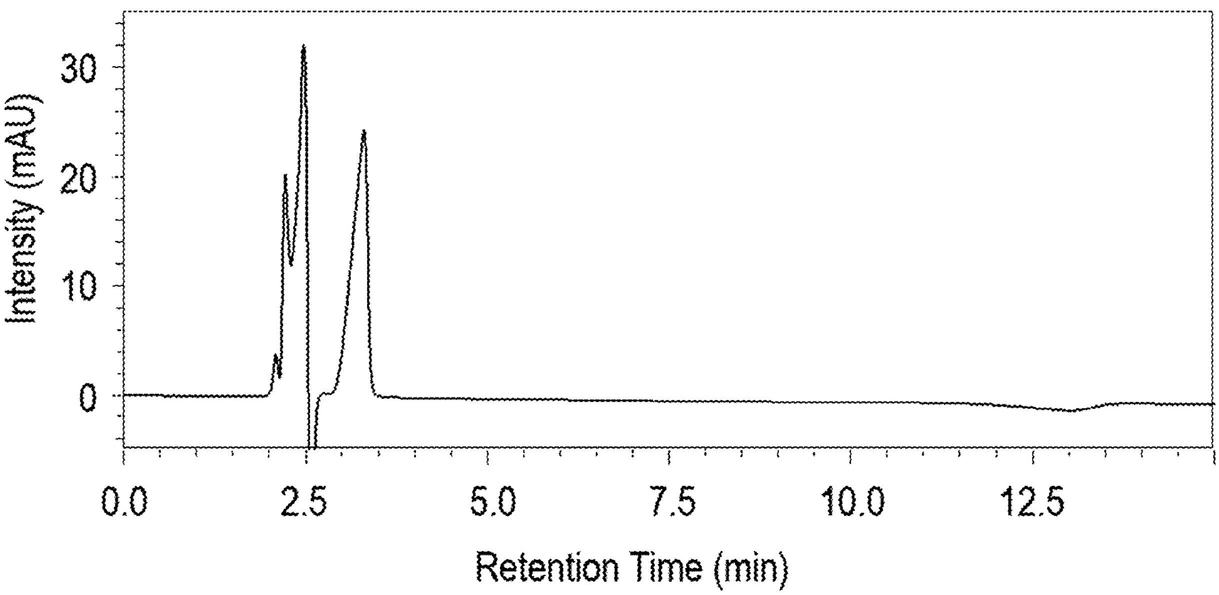
30
20
10
0
Intensity (mAU)
0.0
2.5
5.0
7.5
10.0
12.5
Retention Time (min)

FIG. 10

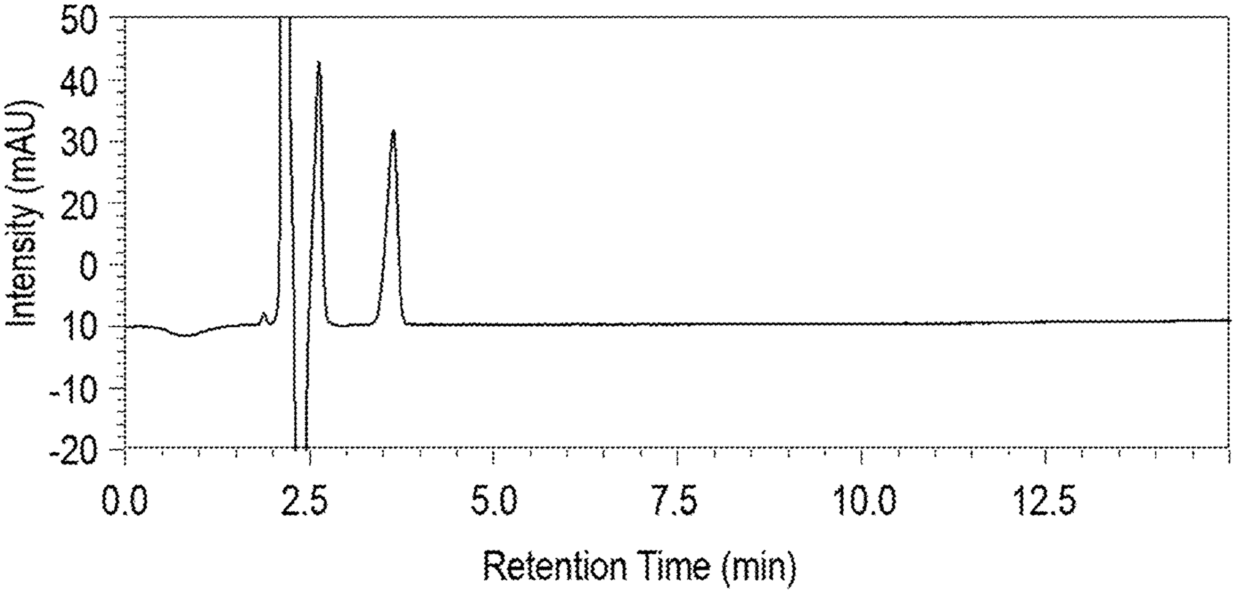

50
40
30
20
0
10
-10
-20
Intensity (mAU)
0.0
2.5
5.0
7.5
10.0
12.5
Retention Time (min)


FIG. 11

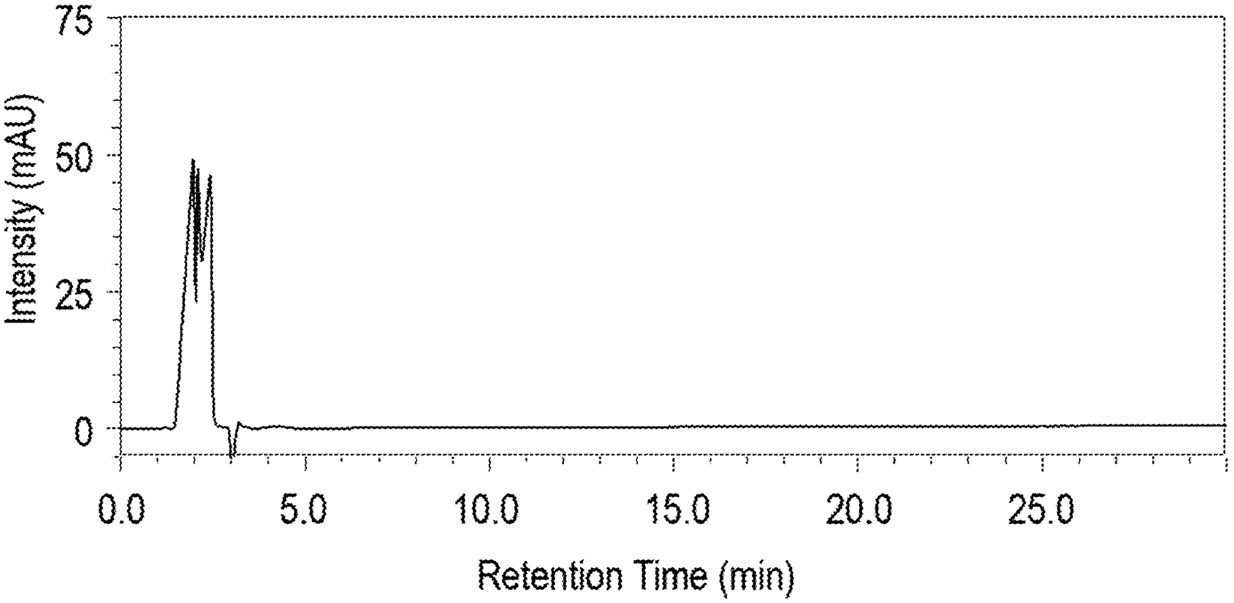

75
50
25
0
Intensity (mAU)
0.0
5.0
10.0
15.0
20.0
25.0
Retention Time (min)


FIG. 12

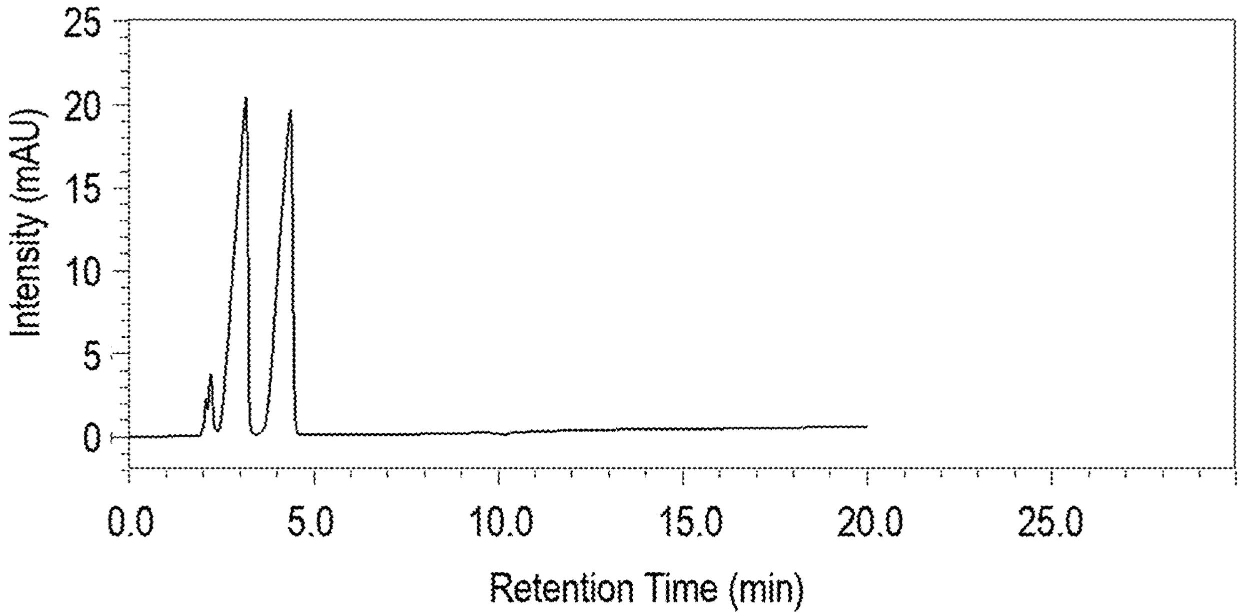
Intensity (mAU)
25
20
15
10
5
0
0.0
5.0
10.0
15.0
20.0
25.0
Retention Time (min)

FIG. 13

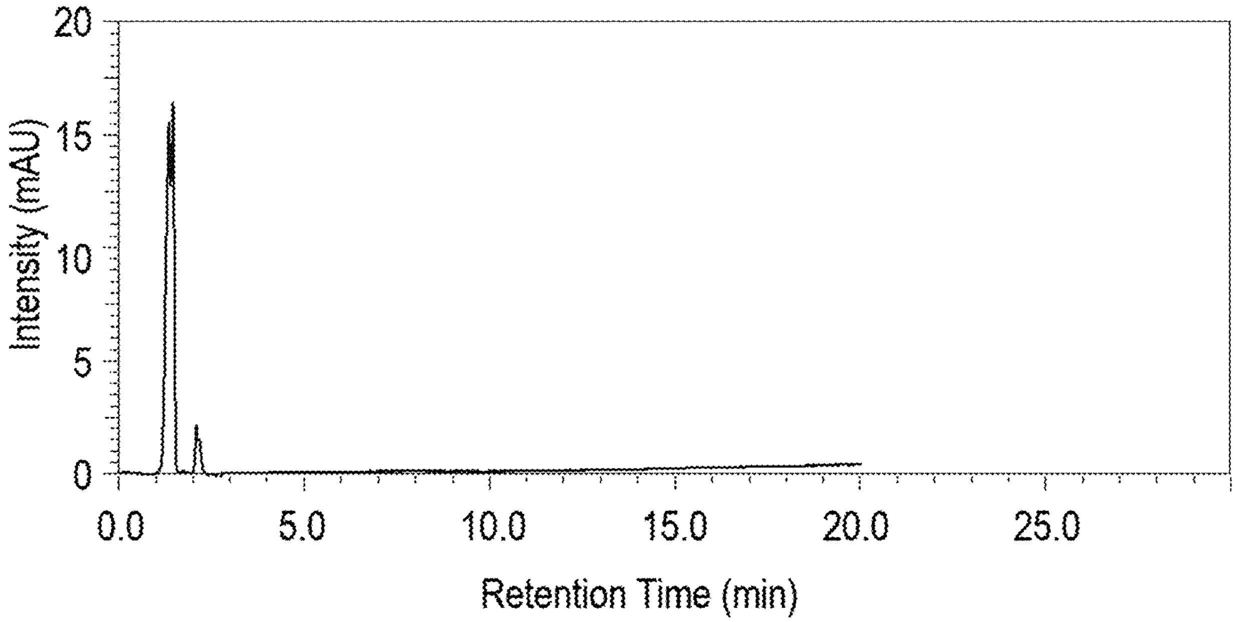
Intensity (mAU)
20
15
10
5
0
0.0
5.0
10.0
15.0
20.0
25.0
Retention Time (min)

FIG. 14

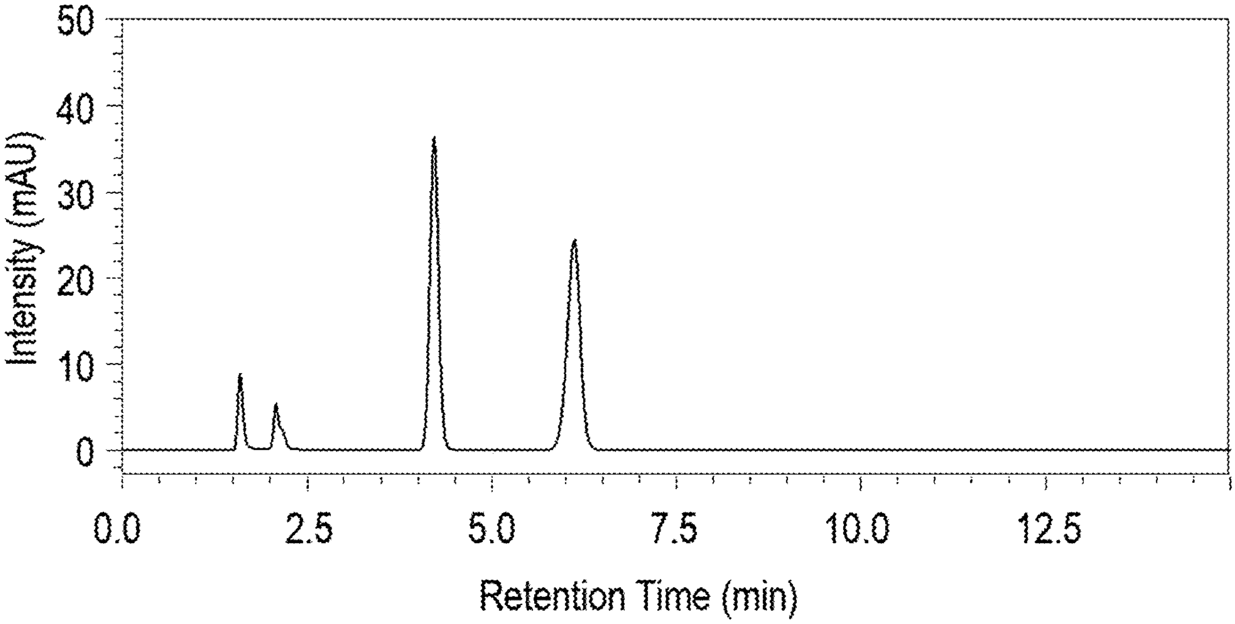
50
40
30
20
10
0
Intensity (mAU)
0.0
2.5
5.0
7.5
10.0
12.5
Retention Time (min)

FIG. 15

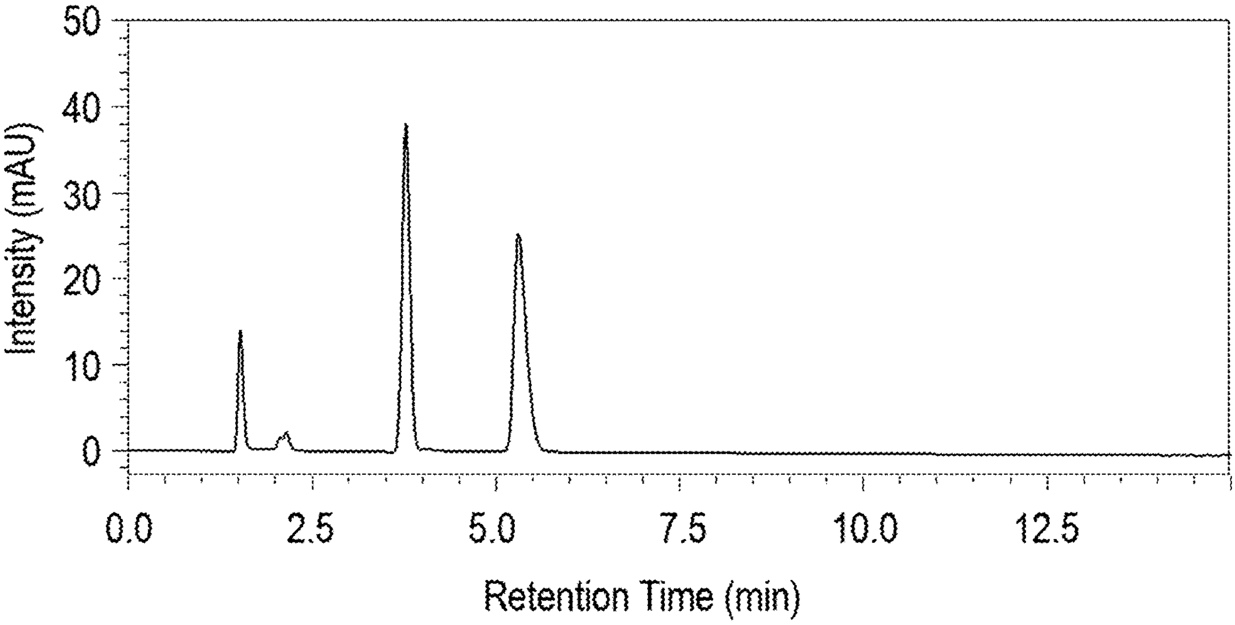
50
40
30
20
10
0
Intensity (mAU)
0.0
2.5
5.0
7.5
10.0
12.5
Retention Time (min)

FIG. 16

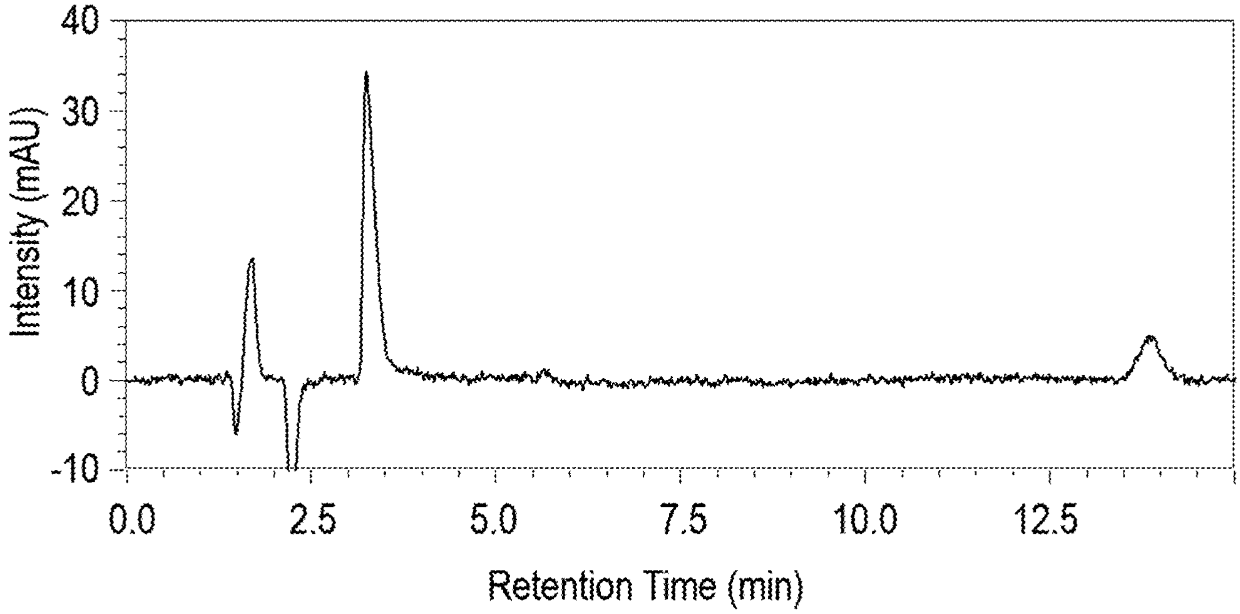
40
30
20
10
0
-10
Intensity (mAU)
0.0
2.5
5.0
7.5
10.0
12.5
Retention Time (min)

FIG. 17

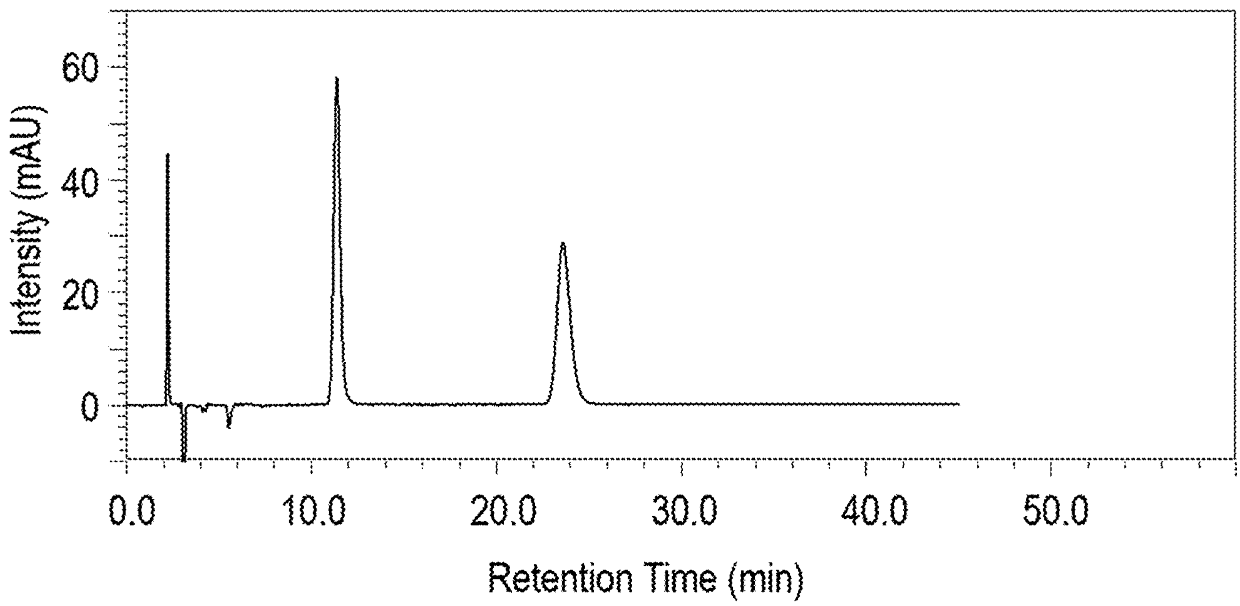
60
40
20
0
Intensity (mAU)
0.0
10.0
20.0
30.0
40.0
50.0
Retention Time (min)

FIG. 18

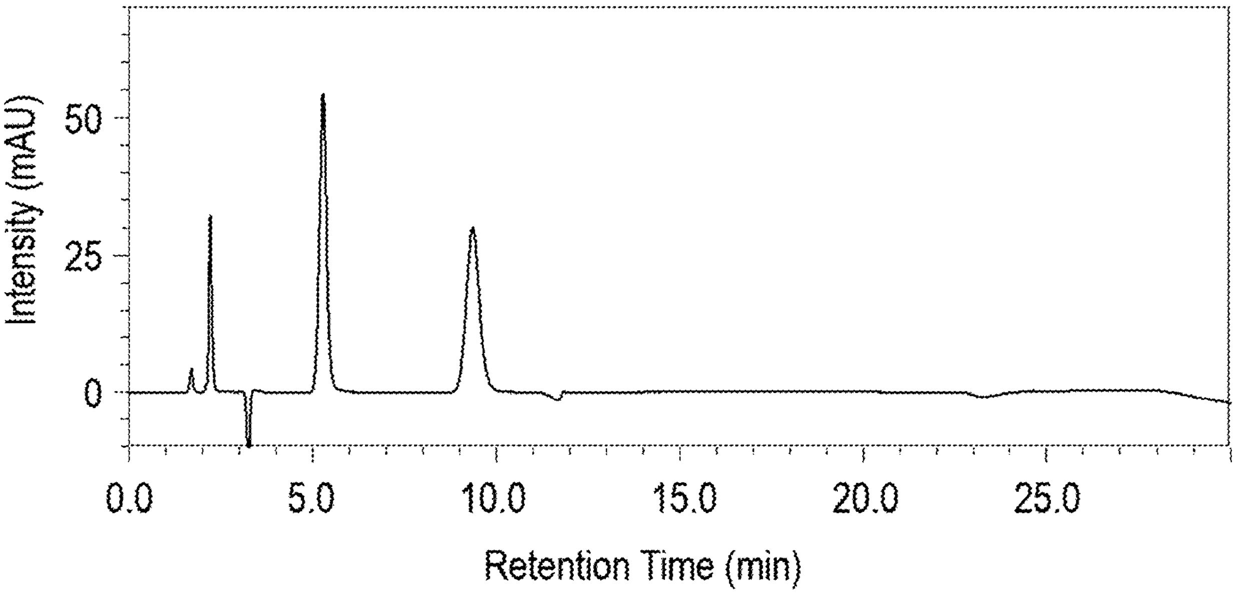
Intensity (mAU)
50
25
0
0.0
5.0
10.0
15.0
20.0
25.0
Retention Time (min)

FIG. 19

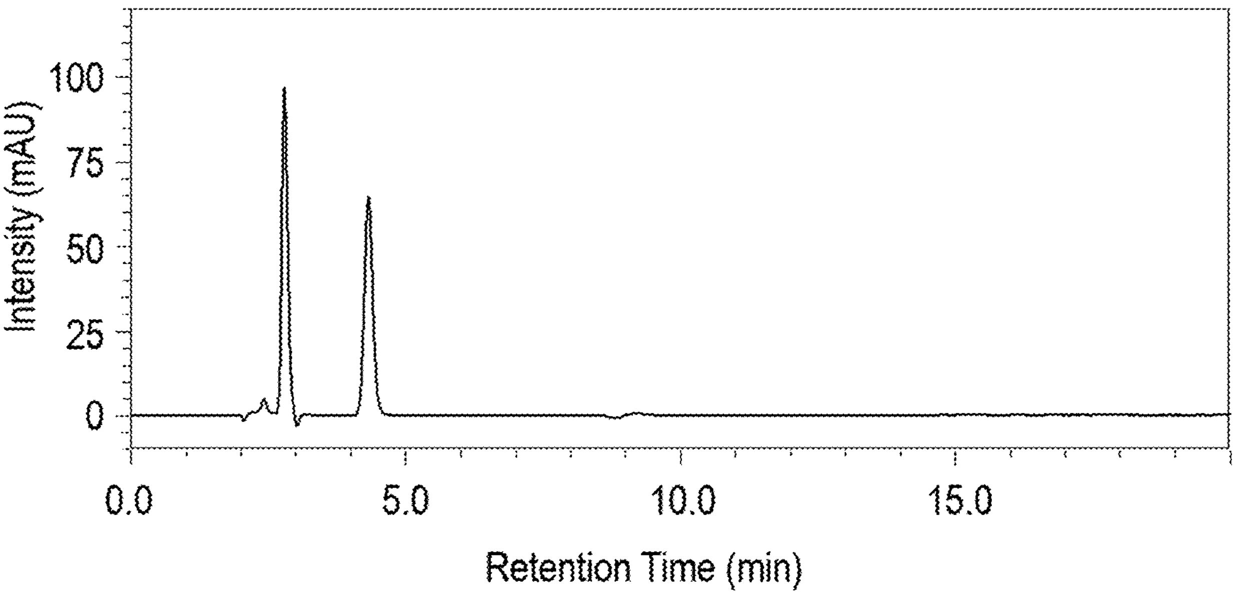
Intensity (mAU)
100
75
50
25
0
0.0
5.0
10.0
15.0
Retention Time (min)

FIG. 20

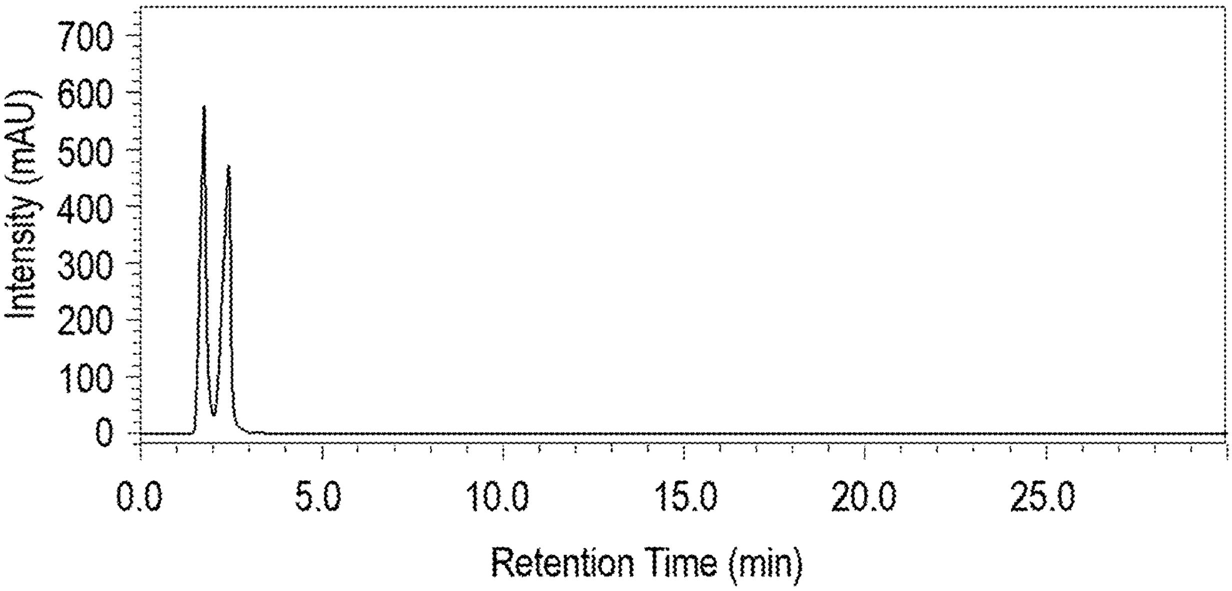
700
600
500
400
300
200
100
0
Intensity (mAU)
0.0
5.0
10.0
15.0
20.0
25.0
Retention Time (min)

FIG. 21

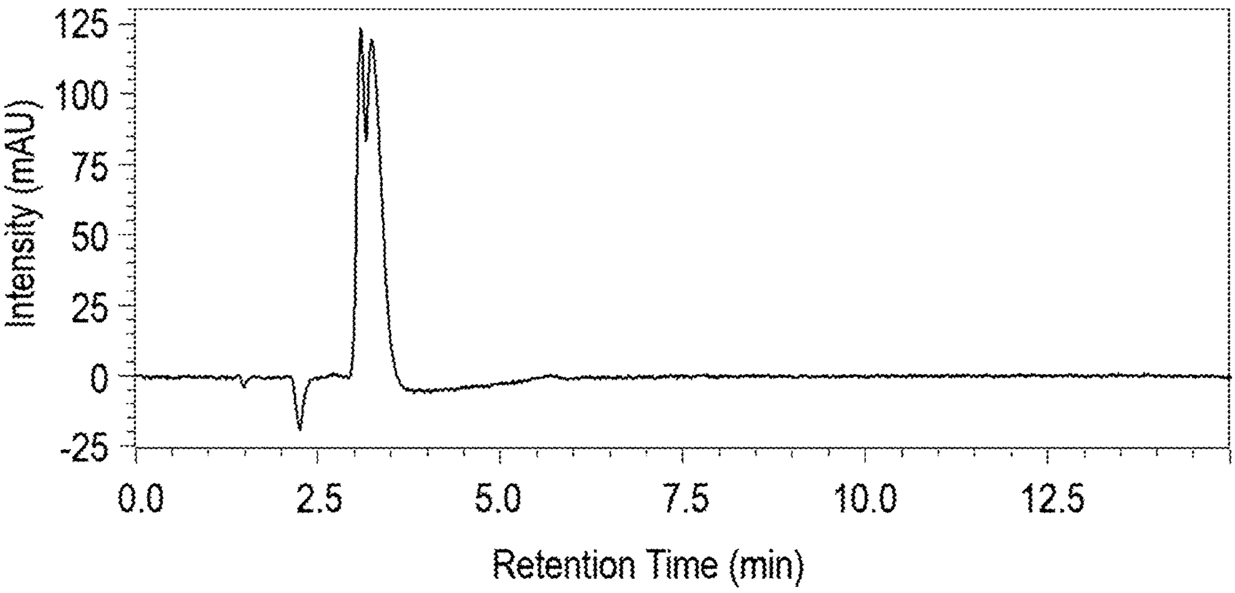
125
100
75
50
25
0
-25
Intensity (mAU)
0.0
2.5
5.0
7.5
10.0
12.5
Retention Time (min)

FIG. 22

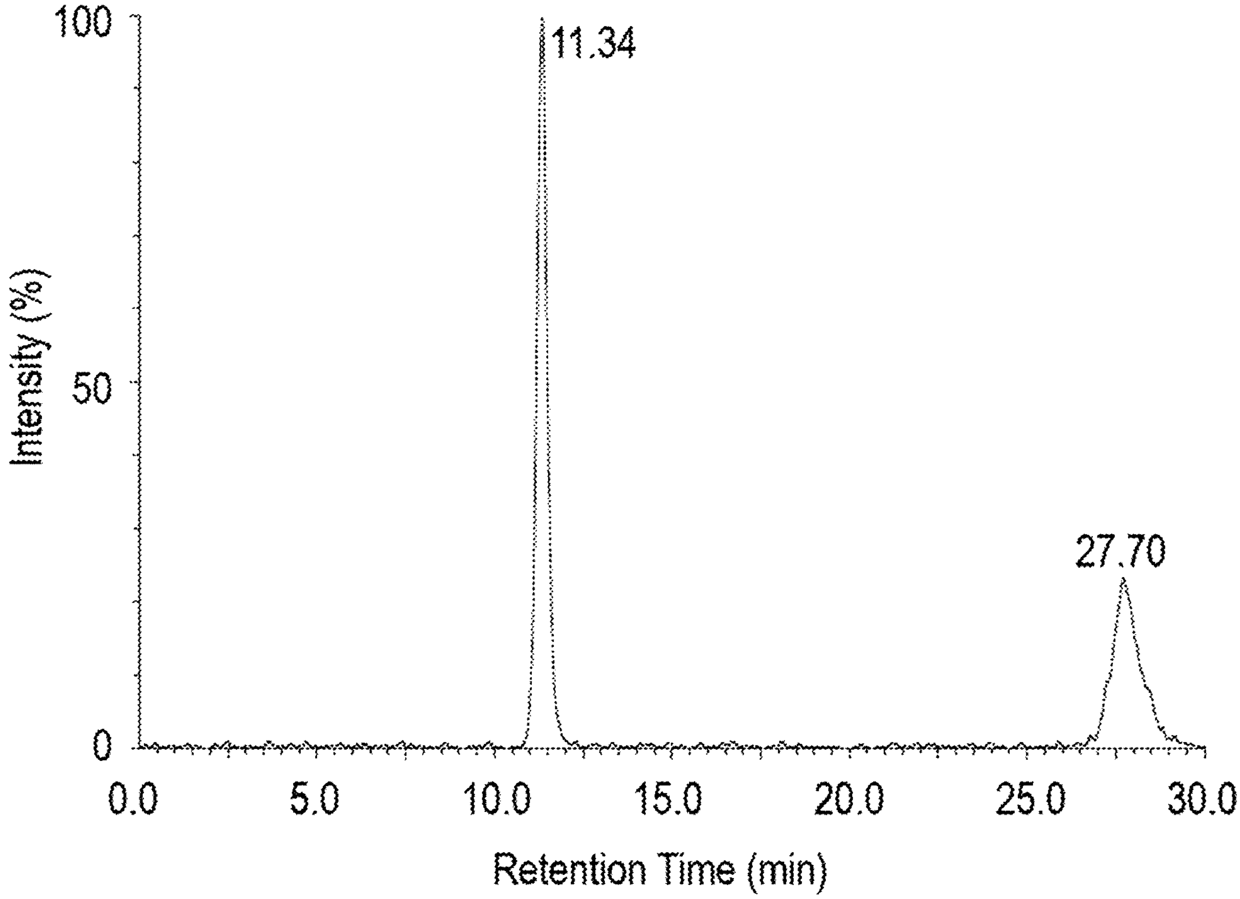
100
50
0
Intensity (%)
11.34
27.70
0.0
5.0
10.0
15.0
20.0
25.0
30.0
Retention Time (min)

FIG. 23

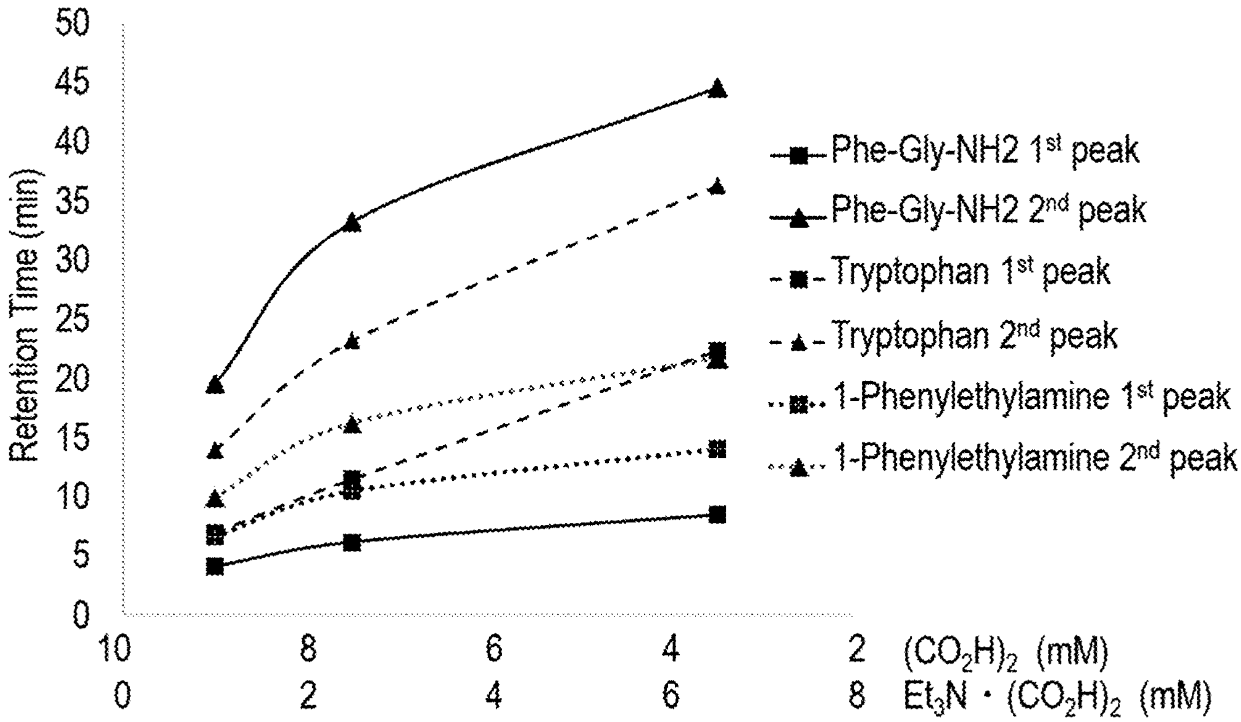

Retention Time (min)
50
45
40
35
30
25
20
15
10
5
0
10
8
6
4
2
(CO2H)2 (mM)
0
2
4
6
8
Et3N · (CO2H)2 (mM)
Phe-Gly-NH2 1st peak
Phe-Gly-NH2 2nd peak
Tryptophan 1st peak
Tryptophan 2nd peak
1-Phenylethylamine 1st peak
1-Phenylethylamine 2nd peak


FIG. 24

**INTERNATIONAL SEARCH REPORT**

International application No. **PCT/JP2023/030453**

**A. CLASSIFICATION OF SUBJECT MATTER**

***C07C 209/88***(2006.01)i; ***B01D 15/16***(2006.01)i; ***B01D 15/38***(2006.01)i; ***B01D 15/40***(2006.01)i; ***B01J 20/29***(2006.01)i; ***C07B 57/00***(2006.01)i; ***C07C 211/27***(2006.01)i; ***C07D 209/20***(2006.01)i; ***G01N 30/72***(2006.01)i
FI: C07C209/88; C07C211/27; C07D209/20; B01D15/38; B01D15/40; B01D15/16; B01J20/29; G01N30/72 C; C07B57/00 360

According to International Patent Classification (IPC) or to both national classification and IPC

**B. FIELDS SEARCHED**

Minimum documentation searched (classification system followed by classification symbols)

C07C209/88; B01D15/16; B01D15/38; B01D15/40; B01J20/29; C07B57/00; C07C211/27; C07D209/20; G01N30/72

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | HYUN, Myung-Ho et al. Liquid chromatographic resolution of racemic amines, amino alcohols and related compounds on a chiral crown ether stationary phase. JOURNAL OF CHROMATOGRAPHY A. 2002, 959, pp. 75-83<br>p. 76, right column, line 3 to p. 78, left column | 1–12 |
| X | HYUN, Myung-Ho et al. New chiral crown ether stationary phase for the liquid chromatographic resolution of α-amino acid enantiomers. JOURNAL OF CHROMATOGRAPHY A. 2001, 910, pp. 359-365<br>table 4, fig. 1 | 1-12 |
| X | WO 2020/251003 A1 (DAICEL CORP) 17 December 2020 (2020-12-17)<br>example 7, paragraphs [0022], [0043] | 1-8, 10-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 November 2023** | **14 November 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2023/030453**

| Patent document cited in search report | Publication date (day/month/year) | Patent family member(s) | Publication date (day/month/year) |
|---|---|---|---|
| WO 2020/251003 A1 | 17 December 2020 | US 2022/0339555 A1<br>example 7, paragraphs [0065], [0108]<br>EP 3984615 A1<br>CN 113905797 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H269472 A
- WO 2012050124 A
- WO 2020251003 A
- JP 2014169259 A


### Non-patent literature cited in the description


- **KONYA Y** ; **TANIGUCHI M** ; **FURUNO M** ; **NAKANO Y** ; **TANAKA N** ; **FUKUSAKI E**. *Journal of Chromatography A*, 2018, vol. 1578, 35-44
- **PENG WU**. *Chinese Journal of Chemistry*, 2017, vol. 35, 1037-1042